# EUROPEAN PATENT APPLICATION

(11) **EP 4 806 919 A1**
(43) Date of publication of application: **16.09.2026**
(21) Application number: 24888057.7
(22) Date of filing: 07.11.2024
(51) Int. Cl.: C07K 16/28, C07K 19/00, C12N 15/62, C12N 5/10, C12N 15/13, A61K 39/395

(54) **ANTI-BCMA SINGLE-DOMAIN ANTIBODY AND USE THEREOF**

(30) Priority: 07.11.2023 CN 202311471905
(71) Applicant: Cells & Genes Biotech (Shanghai) Co., Ltd, Shanghai 201203 (CN)
(72) Inventor: LI, Linhong, Shanghai 201203 (CN); ZHU, Xiangyang, Shanghai 201203 (CN); XIN, Pengcheng, Shanghai 201203 (CN); WANG, Li, Shanghai 201203 (CN); YU, Shijun, Shanghai 201203 (CN); YANG, Xingxing, Shanghai 201203 (CN); WANG, Chengcheng, Shanghai 201203 (CN)
(74) Representative: Santoro, Sofia
(86) International application number: PCT/CN2024/130558
(87) International publication number: WO 2025/098446

(57) **Abstract**

Provided are an anti-BCMA single-domain antibody and a use thereof. Specifically, provided is a single-domain antibody specifically targeting BCMA, having high affinity for BCMA and can bind to BCMA of human or monkey origin. Also provided are a CAR cell comprising the single-domain antibody and a treatment method.

## Description

### TECHNICAL FIELD

The present invention relates to the field of biomedicine. Specifically, the present invention relates to anti-BCMA single domain antibody and use thereof.

### BACKGROUND

Multiple myeloma (MM) is a tumor derived from the malignant transformation of B lymphocyte-derived plasma cells in bone marrow. Symptoms of MM may include bone pain (often in the back or ribs), fever, fatigue, anemia, frequent infections, and easy bruising or bleeding. Treatment may involve various chemotherapies and targeted therapy drugs, including: proteasome inhibitors, immunomodulatory drugs, and monoclonal antibodies, radiotherapy, and stem cell transplantation, etc.. However, MM remains an incurable disease, and most patients eventually relapse, so its treatment still faces significant challenges. The recent two CAR-T cell therapies approved by FDA and one approved in China for the treatment of MM represent a breakthrough in MM treatment. However, MM treatment is still limited due to costs and production in this field, and new therapeutic products and treatment are still in need.

Therefore, there is a need in the art for multiple myeloma therapeutic antibodies with improved therapeutic effects and uses thereof.

### SUMMARY OF THE INVENTION

The object of the present invention is to provide an anti-BCMA single domain antibody and use thereof.

In the first aspect of the present invention, it provides an anti-BCMA single domain antibody, wherein the VHH chain of the single domain antibody has complementarity determining regions CDR1, CDR2, and CDR3 of the heavy chain variable region as set forth in SEQ ID NO: 1, and the complementarity determining regions CDR1, CDR2, and CDR3 are defined by Chothia, Abm, Kabat, or IMGT rule.

In another preferred embodiment, the CDR1, CDR2, and CDR3 are selected from the group consisting of:
(1) on the basis of Chothia rule:
   CDR1 as set forth in SEQ ID NO: 2,
   CDR2 as set forth in SEQ ID NO: 3, and
   CDR3 as set forth in SEQ ID NO: 4; or,
(2) on the basis of Kabat rule:
   CDR1 as set forth in SEQ ID NO: 5,
   CDR2 as set forth in SEQ ID NO: 6, and
   CDR3 as set forth in SEQ ID NO: 4; or,
(3) on the basis of IMGT rule:
   CDR1 as set forth in SEQ ID NO: 7,
   CDR2 as set forth in SEQ ID NO: 8, and
   CDR3 as set forth in SEQ ID NO: 9;
   and, any one of the above CDR sequences further comprises a derivative sequence that is optionally added, deleted, modified and/or substituted with at least one amino acid, and enables the derivative antibody formed by the heavy chain and light chain comprising the derivative CDR sequence to retain the binding affinity to BCMA.

In another preferred embodiment, the number of added, deleted, modified and/or substituted amino acids is 1-3, preferably 1-2, and more preferably 1.

In another preferred embodiment, the VHH chain of the single domain antibody further comprises framework region (FR).

In another preferred embodiment, the CDR1, CDR2, and CDR3 are separated by framework regions FR1, FR2, FR3, and FR4 of the VHH chain.

In another preferred embodiment, the framework region FR is of human, murine, rabbit or camel origin.

In another preferred embodiment, the VHH chain of the single domain antibody has an amino acid sequence having ≥55%, ≥60%, ≥65%, ≥70%, ≥75%, ≥80%, ≥85%, ≥90%, ≥95%, ≥96%, ≥97%, ≥98%, or ≥99% sequence identity to the amino acid sequence as set forth in SEQ ID NO: 1.

In another preferred embodiment, the VHH chain of the single domain antibody has an amino acid sequence as set forth in SEQ ID NO: 1.

In the second aspect of the present invention, it provides an anti-BCMA antibody, wherein the antibody comprises one or more VHH chains of the single domain antibody of the first aspect of the present invention.

In another preferred embodiment, the VHH chain of the single domain antibody has an amino acid sequence as set forth in SEQ ID NO: 1.

In another preferred embodiment, the antibody is a monovalent antibody, a bivalent antibody, and/or a multivalent antibody.

In another preferred embodiment, the antibody is an animal-derived antibody, a humanized antibody, a chimeric antibody, or a chimeric antigen receptor antibody (CAR).

In another preferred embodiment, the CDR region of the humanized antibody comprises 0, 1, 2, or 3 amino acid changes, and the humanized antibody retains ≥70%, preferably ≥80%, more preferably ≥90% of the BCMA binding activity compared with the animal-derived antibody before humanization.

In another preferred embodiment, the animal is a non-human mammal, preferably a mouse, goat, rabbit, or camel.

In another preferred embodiment, the antibody is a double-chain antibody or a single-chain antibody.

In another preferred embodiment, the antibody is a monoclonal antibody.

In another preferred embodiment, the antibody is a partially or fully humanized antibody.

In another preferred embodiment, the antibody further comprises a heavy chain constant region.

In another preferred embodiment, the heavy chain constant region is derived from IgG, preferably human IgG.

In another preferred embodiment, the antibody is a heavy chain antibody.

In another preferred embodiment, any one of the above amino acid sequences further comprises a derivative sequence that is optionally added, deleted, modified and/or substituted with at least one amino acid and can retain the binding affinity to BCMA.

In another preferred embodiment, the number of added, deleted, modified and/or substituted amino acids does not exceed 40%, preferably ≤20%, and more preferably ≤10% of the total number of amino acids in the initial amino acid sequence.

In the third aspect of the present invention, it provides a multispecific antibody, wherein the multispecific antibody comprises: the single domain antibody of the first aspect of the present invention or the anti-BCMA antibody of the second aspect of the present invention.

In another preferred embodiment, the multispecific antibody comprises one or more second antigen binding regions targeting additional antigen targets.

In another preferred embodiment, the second antigen binding region is an antibody or an antibody fragment, wherein the antibody fragment comprises: (i) Fab fragment; (ii) F(ab')2 fragment; (iii) Fd fragment; (iv) Fv fragment; (v) single-chain Fv (scFv) molecule; (vi) dAb fragment.

In another preferred embodiment, the second antigen binding region is a single domain antibody.

In the fourth aspect of the present invention, it provides a recombinant protein, wherein the recombinant protein comprises:
(i) the single domain antibody of the first aspect of the present invention, or the anti-BCMA antibody of the second aspect of the present invention; and
(ii) an optional polypeptide molecule or fragment with therapeutic function; and/or
(iii) an optional functional domain for improving the physicochemical property or druggability of a protein.

In another preferred embodiment, the improving of the physicochemical property or druggability of a protein comprises prolonging the half-life of the anti-BCMA single domain antibody.

In another preferred embodiment, the recombinant protein further comprises: (iv) an optional tag sequence for facilitating expression and/or purification.

In another preferred embodiment, the tag sequence is selected from the group consisting of: 6His tag, GGGS sequence, FLAG tag.

In another preferred embodiment, the recombinant protein is a monomer, dimer, or polymer.

In another preferred embodiment, the polypeptide molecule or fragment with therapeutic function comprises but is not limited to: insulin, IL-2, interferon, calcitonin, GHRH peptide, intestinal peptide analog, albumin, antibody fragment, and cytokine.

In another preferred embodiment, the recombinant protein (or polypeptide) comprises a fusion protein.

In another preferred embodiment, the fusion protein comprises a multispecific antibody, and a chimeric antibody.

In another preferred embodiment, the functional domain for improving the physicochemical property or druggability of a protein comprises an Fc segment, human serum albumin (HSA).

In another preferred embodiment, the VHH chain of the anti-BCMA single domain antibody has an amino acid sequence as set forth in SEQ ID NO: 1.

In the fifth aspect of the present invention, it provides a chimeric antigen receptor (CAR), wherein the nucleotide sequence of the extracellular antigen-binding domain of the CAR encodes the VHH chain of the single domain antibody of the first aspect of the present invention.

In another preferred embodiment, the VHH chain of the single domain antibody has an amino acid sequence having ≥65%, ≥75%, ≥80%, ≥85%, ≥90%, ≥95%, ≥96%, ≥97%, ≥98%, or ≥99% sequence identity to the amino acid sequence as set forth in SEQ ID NO: 1, or has an amino acid sequence as set forth in SEQ ID NO: 1.

In another preferred embodiment, the CAR has a structure as shown in Formula I below:

L-EB-H-TM-Cₙ-IS (I)

wherein,
each "-" independently is a linker peptide or a peptide bond;
L is absent or a signal peptide sequence;
EB is the extracellular antigen-binding domain;
H is absent or a hinge region;
TM is a transmembrane domain;
Cₙ is n tandem co-stimulatory signaling molecules, wherein C represents a co-stimulatory signaling molecule, and n is an integer selected from 1, 2, 3, or 4;
IS is a cytoplasmic signal transduction sequence.

In another preferred embodiment, the L is a signal peptide of a protein selected from the group consisting of: CD8, CD28, GM-CSF, CD4, CD137, SECTM1, and a combination thereof.

In another preferred embodiment, the L is a CD8-derived signal peptide.

In another preferred embodiment, the L has an amino acid sequence as set forth in SEQ ID NO: 14.

In another preferred embodiment, the H is a hinge region of a protein selected from the group consisting of: CD8, CD28, CD137, and a combination thereof.

In another preferred embodiment, the H is a CD8-derived hinge region.

In another preferred embodiment, the TM is a transmembrane region of a protein selected from the group consisting of: CD28, CD3 epsilon, CD45, CD4, CD5, CD8, CD9, CD16, CD22, CD33, CD37, CD64, CD80, CD86, CD134, CD137, CD154, and a combination thereof.

In another preferred embodiment, the TM is a CD8-derived transmembrane region.

In another preferred embodiment, the H-TM fragment has an amino acid sequence as set forth in SEQ ID NO: 16.

In another preferred embodiment, the n co-stimulatory signaling molecules are each independently a co-stimulatory signaling molecule of a protein selected from the group consisting of: OX40, CD2, CD7, CD27, CD28, CD30, CD40, CD70, CD134, 4-1BB(CD137), PD1, Dap10, CDS, ICAM-1, LFA-1 (CD11a/CD18), ICOS (CD278), NKG2D, GITR, TLR2, and a combination thereof.

In another preferred embodiment, n is 1.

In another preferred embodiment, C is a 4-1BB-derived co-stimulatory signaling molecule.

In another preferred embodiment, the cytoplasmic signal transduction sequence is a CD3ζ cytoplasmic signaling sequence.

In another preferred embodiment, the Cₙ-IS fragment has an amino acid sequence as set forth in SEQ ID NO: 18.

In another preferred embodiment, the CAR has an amino acid sequence as set forth in SEQ ID NO: 25.

In the sixth aspect of the present invention, it provides a polynucleotide, wherein the polynucleotide encodes a polypeptide selected from the group consisting of:
(1) the single domain antibody of the first aspect of the present invention, the anti-BCMA antibody of the second aspect of the present invention, or the multispecific antibody of the third aspect of the present invention;
(2) the recombinant protein of the fourth aspect of the present invention; and
(3) the CAR of the fifth aspect of the present invention.

In another preferred embodiment, the polynucleotide comprises RNA, DNA or cDNA.

In another preferred embodiment, the polynucleotide further comprises a sequence encoding an additional functional protein selected from the group consisting of: IL-21 or a functionally active fragment thereof, IL-15 or a functionally active fragment thereof, IL-2 or a functionally active fragment thereof, IL-7 or a functionally active fragment thereof, IL-10 or a functional fragment thereof, a cytokine, an immune blocker targeting PD1, TIGIT, VGEF, CTLA-4, CD73, an antibody, a bispecific T-cell engager (BiTE), and a combination thereof.

In another preferred embodiment, the polynucleotide comprises a sequence encoding IL-15 and/or IL-21.

In another preferred embodiment, the polynucleotide comprises a left homology arm and a right homology arm, wherein the homology arms are used to integrate the polynucleotide into a host cell.

In another preferred embodiment, the nucleic acid molecule comprises tandem expression units, comprising:
(E1) a first expression unit for expressing the chimeric antigen receptor;
(E2) a second expression unit for expressing IL-15, or a functionally active fragment or fusion protein thereof;
(E3) a third expression unit for expressing IL-21, or a functionally active fragment or fusion protein thereof; and
(E4) optionally, a fourth expression unit for expressing the additional functional protein,
wherein the positions of the first, second, third and fourth expression units are arbitrarily interchangeable.

In the nucleic acid molecule, each independent expression unit is driven by an exogenous promoter or an endogenous promoter operably linked thereto, or is driven by an upstream promoter and has a nucleic acid sequence encoding a cleavable moiety (e.g., a 2A nucleic acid sequence, an IRES nucleic acid sequence) at its 5' end.

In another preferred embodiment, the number of the first expression unit, the second expression unit, and the third expression unit is each independently 1, 2, or 3.

In another preferred embodiment, the number of the fourth expression unit is 0, 1, 2, 3, 4, or 5.

In another preferred embodiment, the nucleic acid molecule encodes 1, 2, or 3 identical or different CAR molecules.

In another preferred embodiment, the nucleic acid molecule has a structure of Formula I below:

ARM5-P1-CAR1-P2/L1-Z1-P3/L2-Z2-(P4/L3-Z3)ₘ-ARM3 (Ia )

wherein,
ARM5 is absent or a 5' homology arm;
ARM3 is absent or a 3' homology arm;
P1 is absent of promoter, a splicing acceptor, or a first exogenous promoter;
CAR1 is a nucleic acid sequence encoding the chimeric antigen receptor (CAR) of claim 7;
P2/L1 is a second exogenous promoter P2 or a nucleic acid sequence L1 encoding a cleavable moiety;
Z1 is absent, or a nucleotide sequence encoding a second CAR, or IL-15 or a functional protein fragment thereof;
P3/L2 is absent or a third exogenous promoter P3 or a nucleic acid sequence L2 encoding a cleavable moiety;
Z2 is absent, or a nucleotide sequence encoding IL-21 or a functional protein fragment thereof;
P4/L3 is each independently a fourth exogenous promoter P4 or a nucleic acid sequence L3 encoding a cleavable moiety;
Z3 is a nucleic acid sequence encoding an additional functional protein;
m is 0, 1, 2, 3, 4 or 5.

In another preferred embodiment, the promoters P1, P2, P3, and each P4 are the same or different exogenous promoters, and the cleavable moieties L1, L2, and each L3 are nucleic acid sequences encoding the same or different cleavable moieties.

In another preferred embodiment, the m Z3 are nucleic acid sequences encoding the same or different additional functional proteins.

In another preferred embodiment, ARM5 is a 5' homology arm, and ARM3 is a 3' homology arm.

In another preferred embodiment, the IL-15 has an amino acid sequence as set forth in SEQ ID NO: 20.

In another preferred embodiment, the IL-21 has an amino acid sequence as set forth in SEQ ID NO: 21.

In another preferred embodiment, the exogenous promoter is a PGK promoter or an EF1a promoter.

In another preferred embodiment, the PGK promoter has a nucleotide sequence as set forth in SEQ ID NO: 23.

In another preferred embodiment, the EF1a promoter has a nucleotide sequence as set forth in SEQ ID NO: 26.

In another preferred embodiment, the functional protein is selected from the group consisting of: IL-21 or a functionally active fragment thereof, IL-15 or a functionally active fragment thereof, IL-2 or a functionally active fragment thereof, IL-7 or a functionally active fragment thereof, IL-10 or a functional fragment thereof, a cytokine, an immune blocker targeting PD1, TIGIT, VGEF, CTLA-4, CD73, an antibody, a BiTE, and a combination thereof.

In another preferred embodiment, the polynucleotide has a nucleotide sequence as set forth in SEQ ID NO: 10, or has ≥85%, ≥90%, ≥95%, ≥96%, ≥97%, ≥98%, or ≥99% sequence identity to SEQ ID NO: 10.

In the seventh aspect of the present invention, it provides a vector, wherein the vector comprises the polynucleotide of the sixth aspect of the present invention.

In another preferred embodiment, the vector comprises: a bacterial plasmid, a phage, a yeast plasmid, a plant cell virus, a mammalian cell virus such as an adenovirus, retrovirus, or other vector.

In another preferred embodiment, the vector is a plasmid.

In another preferred embodiment, the plasmid is introduced into a host cell by electroporation.

In the eighth aspect of the present invention, it provides a host cell, wherein the host cell comprises the vector of the seventh aspect of the present invention or has the polynucleotide of the sixth aspect of the present invention integrated into its genome.

In the ninth aspect of the present invention, it provides a recombinant immune cell, wherein the immune cell expresses exogenously the CAR of the fifth aspect of the present invention, comprises the vector of the seventh aspect of the present invention, or has the polynucleotide of the sixth aspect of the present invention integrated into its genome.

In another preferred embodiment, the immune cell is selected from a NK cell or a T cell.

In another preferred embodiment, the immune cell is from human or non-human mammal (such as mouse).

In another preferred embodiment, the immune cell further expresses a functional protein selected from the group consisting of: IL-21 or a functionally active fragment thereof, IL-15 or a functionally active fragment thereof, IL-2 or a functionally active fragment thereof, IL-7 or a functionally active fragment thereof, IL-10 or a functional fragment thereof, a cytokine, an immune blocker targeting PD1, TIGIT, VGEF, CTLA-4, CD73, an antibody, a BiTE, and a combination thereof.

In another preferred embodiment, the polynucleotide is introduced into the immune cell by plasmid electroporation method.

In another preferred embodiment, the polynucleotide is site-specifically integrated into the DNA of the immune cell by plasmid electroporation and a site-directed nuclease method.

In another preferred embodiment, the site-directed nuclease comprises a CRISPR-associated protein and an sgRNA.

In another preferred embodiment, the site-directed nuclease comprises an sgRNA targeting TRAC gene.

In another preferred embodiment, the polynucleotide is inserted into the TRAC gene of the immune cell.

In another preferred embodiment, one or more endogenous genes of the immune cell are knocked out.

In another preferred embodiment, the endogenous gene is TRAC.

In another preferred embodiment, the immune cell comprises a polynucleotide having a nucleotide sequence as set forth in SEQ ID NO: 10, or a polynucleotide having ≥85%, ≥90%, ≥95%, ≥96%, ≥97%, ≥98%, or ≥99% identity to SEQ ID NO: 10.

In the tenth aspect of the present invention, it provides an immunoconjugate, wherein the immunoconjugate comprises:
(a) an antibody moiety, wherein the antibody moiety is the single domain antibody of the first aspect of the present invention or the anti-BCMA antibody of the second aspect of the present invention; and
(b) a coupling moiety coupled to the single domain antibody moiety, wherein the coupling moiety is selected from the group consisting of: a detectable label, a drug, a toxin, a cytokine, an enzyme, and a combination thereof.

In another preferred embodiment, the immunoconjugate is a single domain antibody-drug conjugate.

In another preferred embodiment, the single domain antibody moiety is coupled to the coupling moiety via a chemical bond or a linker.

In another preferred embodiment, the coupling moiety is a chemical label or a biological label.

In another preferred embodiment, the chemical label is an isotope, an immunotoxin and/or a chemical drug.

In another preferred embodiment, the biological label is biotin, avidin or an enzyme label.

In another preferred embodiment, the drug is a cytotoxic drug.

In another preferred embodiment, the detectable label comprises a radionuclide, and the radionuclide comprises:
(i) a diagnostic isotope selected from the group consisting of: Tc-99m, Ga-68, F-18, I-123, I-125, I-131, In-111, Ga-67, Cu-64, Zr-89, C-11, Lu-177, Re-188, and a combination thereof; and/or
(ii) a therapeutic isotope selected from the group consisting of: Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133, Yb-169, Yb-177, and a combination thereof.

In another preferred embodiment, the immunoconjugate comprises: a multivalent (such as bivalent) single domain antibody of the first aspect of the present invention or anti-BCMA antibody of the second aspect of the present invention.

In another preferred embodiment, the "multivalent" means that the amino acid sequence of the immunoconjugate comprises multiple repeats of the single domain antibody of the first aspect of the present invention or the anti-BCMA antibody of the second aspect of the present invention.

In another preferred embodiment, the detection is *in vivo* detection or *in vitro* detection.

In another preferred embodiment, the immunoconjugate is used for diagnosing and/or treating a tumor that expresses BCMA protein.

In another preferred embodiment, the immunoconjugate has the molecular formula as follows: 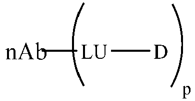 wherein:
nAb is the anti-BCMA single domain antibody, the anti-BCMA antibody, or the multispecific antibody;
LU is a linker (also called a connector);
D is a drug;
the subscript p is a value selected from 1-10.

In the eleventh aspect of the present invention, it provides a pharmaceutical composition comprising:
(i) the single domain antibody of the first aspect of the present invention, the anti-BCMA antibody of the second aspect of the present invention, the multispecific antibody of the third aspect of the present invention, the recombinant protein of the fourth aspect of the present invention, the recombinant immune cell of the ninth aspect of the present invention, or the immunoconjugate of the tenth aspect of the present invention; and
(ii) a pharmaceutically acceptable carrier.

In another preferred embodiment, the pharmaceutical composition further comprises other biologically active substance, such as a drug for treating a tumor.

In another preferred embodiment, the administration method of the pharmaceutical composition is selected from the group consisting of: subcutaneous injection, intradermal injection, intramuscular injection, intravenous injection, intraperitoneal injection, microneedle injection, oral administration, or oral and nasal spraying and aerosol inhalation.

In another preferred embodiment, the dosage form of the pharmaceutical composition is selected from the group consisting of: liquid, solid, and gel.

In another preferred embodiment, the pharmaceutical composition is a liquid formulation.

In another preferred embodiment, the pharmaceutical composition is an injection.

In the twelfth aspect of the present invention, it provides a use of an active ingredient, wherein the active ingredient is selected from the group consisting of: the single domain antibody of the first aspect of the present invention, the anti-BCMA antibody of the second aspect of the present invention, the multispecific antibody of the third aspect of the present invention, the recombinant protein of the fourth aspect of the present invention, the recombinant immune cell of the ninth aspect of the present invention, the immunoconjugate of the tenth aspect of the present invention, and a combination thereof, wherein the active ingredient is used for (a) preparing a detection reagent, a detection plate or a kit; and/or (b) preparing a medicine for the prevention and/or treatment of a BCMA-related disease.

In another preferred embodiment, the detection reagent, detection plate or kit is used for:
(1) detecting BCMA protein in a sample; and/or
(2) detecting endogenous BCMA protein in a tumor cell; and/or
(3) detecting a tumor cell expressing BCMA protein.

In another preferred embodiment, the type of detection comprises but is not limited to flow cytometry, cell immunofluorescence detection, enzyme-linked immunosorbent assay, immunoblotting detection, etc.

In another preferred embodiment, the detection reagent, detection plate or kit is used for diagnosing a BCMA-related disease.

In another preferred embodiment, the BCMA-related disease is selected from the group consisting of: cancers.

In another preferred embodiment, the cancer comprises myeloma, for example multiple myeloma.

In the thirteenth aspect of the present invention, it provides a method (including diagnostic or non-diagnostic method) for detecting BCMA in a sample *in vitro,* comprising the steps of:
(1) contacting the sample with the single domain antibody of the first aspect of the present invention, the anti-BCMA antibody of the second aspect of the present invention, or the immunoconjugate of the tenth aspect of the present invention *in vitro;*
(2) detecting whether an antigen-antibody complex is formed, wherein the formation of the complex indicates the presence of BCMA in the sample.

In another preferred embodiment, the detection comprises diagnostic or non-diagnostic detection.

In the fourteenth aspect of the present invention, it provides a method for preparing a recombinant polypeptide, comprising:
(a) culturing the host cell of the eighth aspect of the present invention under conditions suitable for expression;
(b) isolating the recombinant polypeptide from the culture, wherein the recombinant polypeptide is the single domain antibody of the first aspect of the present invention, the anti-BCMA antibody of the second aspect of the present invention, the multispecific antibody of the third aspect of the present invention, or the recombinant protein of the fourth aspect of the present invention.

In the fifteenth aspect of the present invention, it provides a method for treating a BCMA-related disease, comprising: administering to a subject in need thereof the single domain antibody of the first aspect of the present invention, the anti-BCMA antibody of the second aspect of the present invention, the multispecific antibody of the third aspect of the present invention, the recombinant protein of the fourth aspect of the present invention, the recombinant immune cell of the ninth aspect of the present invention, the immunoconjugate of the tenth aspect of the present invention, or the pharmaceutical composition of the eleventh aspect of the present invention, or a combination thereof.

In another preferred embodiment, the method further comprises: administering other drug or treatment method to the subject in need thereof for combined treatment.

In another preferred embodiment, the other drug or treatment method include: anti-tumor immunotherapy drug, tumor targeted drug, tumor chemotherapy drug, or tumor radiotherapy.

It should be understood that within the scope of the present invention, each technical feature of the present invention described above and in the following (such as Examples) may be combined with each other to form a new or preferred technical solution, which is not listed here due to space limitations.

### BRIEF DESCRIPTION OF THE DRAWINGS

The following drawings are used to illustrate specific embodiments of the present invention and are not intended to limit the scope of the present invention as defined by the claims.
Figure 1 shows the nested PCR amplification results of heavy chain antibody (VHH3).
Figure 2 shows the SDS-PAGE electrophoresis result of nanobody BC-HC-15 transfected into 293F cells and transiently expressed.
Figure 3 shows the binding capacity of nanobody BC-HC-15 to BCMA.
Figure 4 shows the test results of nanobody BC-HC-15 binding to BCMA-negative cells K562 and BCMA-positive cells NCI-H929.
Figure 5 shows the CAR molecular structure constructed with nanobody BC-HC-15 and the structure of the CAR construct expression cassette.
Figure 6 shows the expression level of the BC-HC-15 CAR molecule.
Figure 7 shows the specific killing of BCMA-positive cells by BC-HC-15 CAR-T.
Figure 8 shows the expansion of BC-HC-15 CAR-T stimulated by target cell RPMI-8226.
Figure 9 shows the results of BC-HC-15 CAR-T transplanted into the peripheral blood of NCG mice.
Figure 10 shows the therapeutic effect of BC-HC-15 CAR-T on subcutaneous tumors in NCG mice.
Figure 11 shows the therapeutic effect of G5 CAR-T on the RPMI-1640 subcutaneous tumor animal model.

### DETAILED DESCRIPTION

Through extensive and intensive research, the inventors have developed for the first time a single domain antibody specifically targeting BCMA. The single domain antibody of the present invention has specific high affinity for BCMA and can bind to human or monkey BCMA. CAR cells comprising the single domain antibody of the present invention as an extracellular antigen-binding domain have significant tumor killing ability in animal models. The present invention was completed on this basis.

### Terms

To facilitate understanding of the present invention, certain technical and scientific terms are specifically defined below. Unless otherwise clearly defined herein, all other technical and scientific terms used herein have the meanings commonly understood by one of ordinary skill in the art to which this invention belongs. Before describing the present invention, it should be understood that the invention is not limited to the specific methods and experimental conditions described, as such methods and conditions may vary. It should also be understood that the terminology used herein is for the purpose of describing specific embodiments only and is not intended to be limiting, and the scope of the invention will be limited only by the appended claims.

Unless otherwise defined, all technical and scientific terms used herein have the same meaning as commonly understood by one of ordinary skill in the art to which this invention belongs. As used herein, the term "about," when used in reference to a specifically recited value, means that the value may vary by no more than 1% from the recited value. For example, as used herein, the expression "about 100" comprises 99 and 101 and all values in between (e.g., 99.1, 99.2, 99.3, 99.4, etc.).

The three-letter and one-letter codes for amino acids used in the present invention are as described in J. Biol. Chem, 243, p3558 (1968).

As used herein, the term "treatment" refers to administering a therapeutic agent, including an antibody of the present invention against a respiratory syncytial virus fusion protein (preferably a pre-fusion F protein) and a composition thereof internally or externally to a patient, wherein the patient has one or more disease symptoms for which the therapeutic agent is known to have a therapeutic effect. Typically, it is administered in an amount (therapeutically effective amount) effective to alleviate one or more disease symptoms.

As used herein, the term "optional" or "optionally" means that the subsequently described event or circumstance may occur but are not necessarily required to occur. For example, "optionally comprising 1-3 antibody heavy chain variable regions" means that the antibody heavy chain variable region of a specific sequence may be present but are not necessarily required to be present, and may be 1, 2, or 3.

The term "sequence identity" as used herein refers to the degree of identity between two nucleic acid or two amino acid sequences when optimally aligned and compared with appropriate substitutions, insertions or deletions and other mutations. The sequence identity between a sequence described herein and a sequence having identity thereto can be at least 85%, 90%, or 95%, preferably at least 95%. Non-limiting examples include 85%, 86%, 87%, 88%, 89%, 90%, 91%, 92%, 93%, 94%, 95%, 96%, 97%, 98%, 99%, and 100%.

### Antibody

As used herein, the terms "the single domain antibody of the present invention", " the anti-BCMA nanobody of the present invention", and " the BCMA single domain antibody of the present invention" are used interchangeably and refer to a single domain antibody that specifically recognizes and binds to BCMA (including human BCMA) according to the first aspect of the present invention, particularly preferably a single domain antibody whose VHH chain has an amino acid sequence as set forth in SEQ ID NO: 1.

As used herein, the term "antibody" or "immunoglobulin" is a heterotetrameric glycoprotein of about 150,000 Da having the same structural characteristics, which is composed of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by several constant regions. Each light chain has a variable region (VL) at one end and a constant region at the other end; the constant region of a light chain pairs with the first constant region of a heavy chain, and the variable region of a light chain pairs with the variable region of a heavy chain. Special amino acid residues form an interface between the variable regions of a light chain and a heavy chain.

As used herein, the terms "single domain antibody (VHH)" and "nanobody" have the same meaning and are used interchangeably to refer to the variable region of a monoclonal antibody heavy chain, and constructing a single domain antibody (VHH) composed of only one heavy chain variable region, which is the smallest antigen-binding fragment with complete function. Usually, an antibody that naturally lacks a light chain and a heavy chain constant region 1 (CH1) is obtained first, and then the variable region of the antibody heavy chain is cloned to construct a nanobody (VHH) composed of only one heavy chain variable region.

As used herein, the term "heavy chain antibody" refers to an antibody that contains only a heavy chain. A portion of antibodies found in the blood of camelids are "heavy chain antibodies" that lack light chains. The heavy chain antibody of the present invention comprises a heavy chain variable region (VHH) and heavy chain constant regions CH2 and CH3. The heavy chain antibody of the present invention can be an antibody isolated from an animal (e.g., camel) that naturally lacks light chains and a heavy chain constant region 1 (CH1); or it can be a recombinant antibody obtained by recombining the single domain antibody (VHH) of the present invention with a heavy chain constant region. The heavy chain antibody of the present invention may comprise a constant region derived from, for example, IgG1, IgG2, IgG3, or IgG4, preferably derived from the constant region of IgG1.

As used herein, the term "variable" means that certain parts of the variable region in an antibody differ in sequence, which is responsible for the binding and specificity of various specific antibodies to their specific antigens. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)). The constant regions are not directly involved in the binding of the antibody to the antigen, however, they exhibit different effector functions, for example, involving in the antibody-dependent cytotoxicity of an antibody.

As known to those skilled in the art, immunoconjugates and fusion expression products include: conjugates formed by combining the antibody or a fragment thereof of the present invention with drugs, toxins, cytokines, radionuclides, enzymes, and other diagnostic or therapeutic molecules. The present invention also comprises a cell surface marker or antigen that binds to the anti-BCMA protein antibody or a fragment thereof.

As used herein, the terms "heavy chain variable region" and "VH" are used interchangeably.

As used herein, the term "hypervariable region" and "complementarity determining region (CDR)" are used interchangeably.

In a preferred embodiment of the present invention, the heavy chain variable region of the antibody comprises three complementarity determining regions CDR1, CDR2, and CDR3.

In a preferred embodiment of the present invention, the heavy chain of the antibody comprises the above-mentioned heavy chain variable region and heavy chain constant region.

In the present invention, the terms "the recombinant protein of the present invention", "the protein of the present invention", or "the polypeptide of the present invention" are used interchangeably, and all refer to a polypeptide that specifically binds to BCMA protein, such as a protein or polypeptide having a VHH chain of the single domain antibody of the present invention. They may or may not contain a starting methionine.

The present invention further provides other proteins or fusion expression products having the antibody of the present invention. Specifically, the present invention comprises any protein or protein conjugate and fusion expression product (i.e., immunoconjugate and fusion expression product) having a heavy chain comprising a variable region, as long as the variable region is identical to or at least 90% homologous, preferably at least 95% homologous to the heavy chain variable region of the antibody of the present invention.

In general, the antigen-binding properties of an antibody can be described by three specific regions located in the variable region of the heavy chain, referred as hypervariable regions (CDRs), and the segment is divided into four framework regions (FRs). The amino acid sequences of four FRs are relatively conservative and do not directly involve in binding reactions. These CDRs form a loop structure in which the β-sheets formed by the FRs in between are spatially close to each other, and the CDRs on the heavy chain and the CDRs on the corresponding light chain constitute the antigen-binding site of the antibody. The amino acid sequences of the same type of antibodies can be compared to determine which amino acids constitute the FR or CDR regions.

The variable region of the heavy chain of the antibody of the present invention is of particular interest because at least part of it is involved in binding to the antigen. Thus, the present invention comprises those molecules having an antibody heavy chain variable region with CDRs, provided that their CDRs are 90% or more (preferably 95% or more, and the most preferably 98% or more) identical to the CDRs identified herein.

As used herein, "Chothia", "Kabat", "IMGT", "AbM" refer to the determination of complementary determining regions (CDRs) under the different assignment systems described above. The assignment systems include, for example: Chothia based on the three-dimensional structure of the antibody and the topology of the CDR loops (Chothia et al. (1989) Nature 342:877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), the International ImmunoGeneTics database (IMGT), and the Chothia definition based on loop structure position.

Unless otherwise specified, in the present invention, the term "CDR" or "CDR sequence" encompasses CDR sequences determined by any of the above methods.

Preferably, any one of the above amino acid sequences further comprises a derivative sequence that is optionally added, deleted, modified and/or substituted with at least one amino acid, and may retain the binding affinity to BCMA.

In another preferred embodiment, the sequence formed by adding, deleting, modifying and/or substituting at least one amino acid sequence is preferably an amino acid sequence having a homology of at least 80%, preferably at least 85%, more preferably at least 90%, and most preferably at least 95%.

The antibody of the present invention may be a double-chain or single-chain antibody, and may be selected from an animal-derived antibody, a chimeric antibody, a human-animal chimeric antibody, preferably a humanized antibody.

The antibody derivative of the present invention may be a single-chain antibody and/or an antibody fragment, such as Fab, Fab', (Fab')2 or other antibody derivatives known in the art, as well as any one or more of IgA, IgD, IgE, IgG and IgM antibodies or other subtypes of antibodies.

Among them, the animal is preferably a mammal, such as a mouse or camel.

In a preferred embodiment, the present invention discloses a variety of camel-derived and humanized nanobodies targeting BCMA with high specificity and high affinity, which only comprise a heavy chain, and the heavy chain comprises heavy chain variable region (VHH) amino acid sequences and optional constant regions CH2, CH3.

### Recombinant Protein (or Fusion Protein)

In the present invention, it further comprises a recombinant protein (or fusion protein) comprising the BCMA single domain antibody of the present invention. A preferred fusion protein is a multispecific antibody (e.g., a bispecific antibody). Preferably, the multispecific antibody comprises one or more second antigen binding regions, or further comprises a third antigen binding region.

The present invention comprises not only intact antibodies but also fragments of immunologically active antibody or fusion proteins formed from antibodies with other sequences. Therefore, the present invention also comprises fragments, derivatives and analogs of the antibodies.

As used herein, the terms "fragment", "derivative", and "analog" refer to a polypeptide that substantially retains the same biological function or activity of an antibody of the invention. Polypeptide fragments, derivatives or analogs of the invention may be (i) polypeptides having one or more conservative or non-conservative amino acid residues (preferably conservative amino acid residues) substitutions, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence or pro-protein sequence or sequence used to purify the polypeptide, or a fusion protein formed with a 6His tag). According to the teachings herein, these fragments, derivatives and analogs are within the scope of well-known to those skilled in the art.

The antibody of the present invention refers to a polypeptide having BCMA protein binding activity and comprising the CDR regions as described above. The term also comprises variant forms of polypeptides having the same function as the antibody of the present invention and comprising the CDR regions as described above. These variants include (but are not limited to): deletion, insertion and/or substitution of one or more (usually 1-10, preferably 1-5, more preferably 1-3, and most preferably 1) amino acids, and addition of one or several (usually within 10, preferably within 5, more preferably within 3) amino acids at the C-terminal and/or N-terminal. For example, in the art, substitutions with amino acids of similar properties generally do not alter the function of the protein. For another example, addition of one or several amino acids at the C-terminal and/or N-terminal usually does not alter the function of the protein. The term also comprises active fragments and active derivatives of the antibody of the present invention.

The variant forms of the polypeptide include homologous sequences, conservative variants, alleles, natural mutants, induced mutants, proteins encoded by DNA capable of hybridizing with the coding DNA of the antibody of the present invention under high or low stringency conditions, and polypeptides or proteins obtained by using anti-serum against the antibody of the present invention.

In the present invention, "conservative variant of the antibody of the present invention" refers to a polypeptide formed by substituting at most 10, preferably at most 8, more preferably at most 5, and most preferably at most 3 amino acids with amino acids of similar properties as compared with the amino acid sequence of the antibody of the present invention. These conservative variant polypeptides are preferably produced by amino acid substitution according to Table A.

**Table A**

| Original residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Chimeric Antigen Receptor (CAR)

The design of CARs has undergone the following process: the first generation of CAR has only one intracellular signaling component, CD3ζ or FcyRI. Because it has only one activation domain in the cell, it can only cause transient T cell proliferation and low level of cytokine secretion, and cannot provide long-term T cell proliferation signals and sustained *in vivo* anti-tumor effects, so it did not achieve good clinical results. The second generation of CAR introduces a co-stimulatory molecule, such as CD28, 4-1BB, OX40, ICOS, on the basis of the original structure. Compared with the first generation CAR, the function is greatly improved, further enhancing the persistence of CAR-T cells and their ability to kill tumor cells. On the basis of the second generation CAR, some new immune co-stimulatory molecules, such as CD27, CD134, are added in tandem to develop third and fourth generation CARs.

The chimeric antigen receptor (CAR) of the present invention comprises an extracellular domain, a transmembrane domain, and an intracellular domain. The extracellular domain comprises a target-specific binding element (also referred to as an antigen binding domain or antigen binding structure domain). The intracellular domain comprises a co-stimulatory signaling region and a ζ chain portion. The co-stimulatory signaling region refers to a portion of the intracellular domain comprising a co-stimulatory molecule. Co-stimulatory molecules are cell surface molecules required for effective response of lymphocytes to antigens, other than antigen receptors or their ligands.

A linker may be incorporated between the extracellular domain and the transmembrane domain of the CAR, or between the cytoplasmic domain and the transmembrane domain of the CAR. As used herein, the term "linker" generally refers to any oligopeptide or polypeptide that serves to connect the transmembrane domain to the extracellular domain or cytoplasmic domain of a polypeptide chain. The linker may comprise 0-300 amino acids, preferably 2 to 100 amino acids, and most preferably 3 to 50 amino acids.

In a preferred embodiment of the present invention, the extracellular domain of the CAR provided comprises an antigen binding domain targeting BCMA. When expressed in T cells, the CAR of the present invention is capable of recognizing antigen based on antigen binding specificity. When it binds to its cognate antigen, it affects tumor cells, causing the tumor cells not to grow, to be induced to die, or to be otherwise affected, and leads to reduction or elimination of tumor burden in a patient. The antigen binding domain is preferably fused to an intracellular domain from one or more of a co-stimulatory molecule and the ζ chain. Preferably, the antigen binding domain is fused to an intracellular domain combining an ICOS and/or 4-1BB signaling domain, and a CD3ζ signaling domain.

In the present invention, the antigen binding domain comprises the anti-BCMA single domain antibody of the present invention.

For the hinge region and transmembrane region (transmembrane domain), the CAR can be designed to include a transmembrane domain fused to the extracellular domain of the CAR. In one embodiment, a transmembrane domain naturally associated with one of the domains in the CAR is used. In some embodiments, the transmembrane domain can be selected, or modified by amino acid substitution, to avoid binding such domains to the transmembrane domains of the same or different surface membrane proteins, thereby minimizing interaction with other members of the receptor complex.

The intracellular domain in the CAR of the present invention comprises the signaling domain of ICOS and/or 4-1BB and the signaling domain of CD3ζ.

### CAR-Immune Cell

In the present invention, a recombinant immune cell is provided, which comprises the chimeric antigen receptor specifically targeting BCMA of the present invention.

The chimeric antigen receptor immune cell of the present invention can be a CAR-T cell, a CAR-NK cell, or a CAR-macrophage. Preferably, the chimeric antigen receptor immune cell of the present invention is a CAR-T cell.

The CAR-immune cell of the present invention may additionally express one or more functional proteins. The functional protein comprises, but is not limited to: IL-21 or a functionally active fragment thereof, IL-15 or a functionally active fragment thereof, IL-2 or a functionally active fragment thereof, IL-7 or a functionally active fragment thereof, IL-10 or a functional fragment thereof, a cytokine, an immune blocker targeting PD1, TIGIT, VGEF, CTLA-4, CD73, an antibody, a BiTE, or a combination thereof. The functional protein may be a fusion protein of the above proteins. In one embodiment, the functional protein is exogenous to the CAR-immune cell of the present invention.

The various functional proteins can be expressed under the regulation of the same promoter or different promoters. For example, the functional protein and the CAR of the present invention can be expressed under the regulation of the same promoter. The various functional proteins can be separated from each other, or the functional protein and the CAR can be separated, by a cleavable moiety. The cleavable moiety may comprise a 2A peptide. The 2A peptide may comprise P2A, T2A, F2A, or E2A.

In the CAR-immune cell of the present invention, the CAR and one or more functional proteins can be expressed under the regulation of an endogenous or exogenous promoter. The exogenous promoter can be a PGK promoter or an EF1a promoter.

The CAR of the present invention can be prepared using a non-viral vector, for example, using a plasmid. One method for introducing the CAR of the present invention into an immune cell comprises introducing the plasmid containing the CAR into the immune cell, for example, is electroporation. The transduction can be combined with a gene editing system, for example, using a site-directed nuclease to site-specifically integrate the nucleic acid molecule containing the CAR into a specific position of the immune cell. Usable gene editing systems include CRISPR/sgRNA. For example, a plasmid containing the CAR-encoding nucleic acid of the present invention and a site-directed nuclease (e.g., CRISPR/sgRNA) can be co-electroporated into the immune cell.

The nucleic acid encoding the CAR of the present invention can be inserted into a specific gene of the immune cell by homologous recombination. The polynucleotide may comprise a left homology arm and a right homology arm, wherein the homology arms are used to integrate the polynucleotide into a host cell.

One or more endogenous genes in the CAR immune cell of the present invention can be knocked out or knocked down. The nucleic acid sequence containing the CAR of the present invention can be knocked into the gene desired to be knocked out. The gene editing system may comprise a guide RNA (sgRNA) targeting the gene to be knocked out. In one embodiment, using a Cas protein and an sgRNA targeting the TRAC gene, the nucleic acid sequence containing the CAR of the present invention is knocked into the TRAC gene locus of the immune cell in a one-step electroporation method, while the TRAC gene of the immune cell is knocked out.

In some embodiments, the CAR-immune cell expresses the chimeric antigen receptor (CAR) of the present invention, IL-15 or a functionally active fragment thereof, and IL-21 or a functionally active fragment thereof. In some embodiments, the immune cell comprises a polynucleotide having a nucleotide sequence as set forth in SEQ ID NO: 10, or a polynucleotide having ≥85%, ≥90%, ≥95%, ≥96%, ≥97%, ≥98%, or ≥99% identity to SEQ ID NO: 10.

### Nucleic Acid

The present invention also provides a polynucleotide molecule encoding the above antibody or fragment thereof, or fusion protein thereof, or chimeric antigen receptor (CAR). The polynucleotide of the present invention may be in the form of DNA or RNA. DNA form comprises cDNA, genomic DNA, or synthetic DNA. DNA may be single-stranded or double-stranded. DNA may be a coding strand or a non-coding strand.

The polynucleotide encoding the mature polypeptide of the present invention comprises: the coding sequence that encodes only the mature polypeptide; the coding sequence of the mature polypeptide and various additional coding sequences; the coding sequence of the mature polypeptide (and optional additional coding sequence) and the non-coding sequence.

The term "polynucleotide encoding a polypeptide" may be a polynucleotide that comprises sequence encoding the polypeptide, or a polynucleotide that also comprises additional coding and/or non-coding sequences.

The present invention also relates to a polynucleotide that hybridizes to the sequence as described above and has at least 50%, preferably at least 70%, and more preferably at least 80% sequence identity between the two sequences. In particular, the present invention relates to a polynucleotide that is hybridizable to the polynucleotide of the present invention under strict conditions. In the present invention, "strict conditions" refer to: (1) hybridization and elution at low ionic strength and high temperature, such as 0.2×SSC, 0.1% SDS, 60°C; or (2) hybridization with a denaturing agent, such as 50%(v/v) formamide, 0.1% calf serum/0.1% Ficoll, 42°C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide.

### Preparation of Antibody

The sequence of the DNA molecule for the antibody of the present invention or a fragment thereof may be obtained by conventional techniques, such as PCR amplification or genomic library screening. Once a relevant sequence is obtained, the relevant sequence can be obtained in bulk using a recombination method. This is usually carried out by cloning the sequence into a vector, transforming a cell with the vector, and then separating the relevant sequence from the expanded host cell by conventional methods.

In addition, a relevant sequence may be synthesized artificially, especially when the fragment is short in length. Usually, several small fragments are synthesized first, and then are linked together to obtain a fragment with a long sequence.

At present, it is possible to obtain a DNA sequence encoding the antibody of the present invention (or fragments thereof, or derivatives thereof) completely by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising the suitable DNA sequence and a suitable promoter or a control sequence. These vectors may be used to transform suitable host cells to enable them to express protein.

The host cell may be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the transfected host cell is cultured. Then, the antibody of the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained may be identified by conventional means. For example, the binding specificity of a monoclonal antibody may be determined by immunoprecipitation or an *in vitro* binding assay (such as radioimmunoassay (RIA) or enzyme-linked immunosorbent assay (ELISA)). The binding affinity of a monoclonal antibody may be determined by, for example, the Scatchard analysis (Munson et al., Anal. Biochem. 107: 220 (1980)).

The antibody according to the present invention may be expressed in a cell or on the cell membrane, or be secreted extracellularly. If necessary, the recombinant protein may be separated and purified by various separation methods according to its physical, chemical, and other properties. These methods are well known to those skilled in the art. The examples of these methods comprise, but are not limited to, conventional renaturation treatment, treatment by protein precipitant (such as salt precipitation), centrifugation, cell lysis by osmosis, ultrasonic treatment, super centrifugation, molecular sieve chromatography (gel chromatography), adsorption chromatography, ion exchange chromatography, high performance liquid chromatography (HPLC), and any other liquid chromatography, and a combination thereof.

### Antibody-Drug Conjugate (ADC)

The present invention also provides an antibody-drug conjugate (ADC) based on the antibody of the present invention.

Typically, the antibody-drug conjugate comprises the antibody and an effector molecule, wherein the antibody is conjugated to the effector molecule, and preferably chemical conjugation. Among them, the effector molecule is preferably a drug with therapeutic activity. In addition, the effector molecule may be one or more of a toxic protein, a chemotherapeutic drug, a small-molecule drug or a radionuclide.

The antibody of the present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that links an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly (ethylene glycol)) and therapeutic agents. An antibody may be conjugated to a functional agent to form a conjugate of the antibody-functional agent. A functional agent (e.g., a drug, a detection reagent, a stabilizer) is conjugated (covalently linked) to an antibody. A functional agent may be linked to an antibody either directly or indirectly via a linker.

An antibody may be conjugated to a drug to form an antibody-drug conjugate (ADC). Typically, an ADC comprises a linker between a drug and an antibody. The linker may be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that may be degraded by protease (e.g., lysosomal protease or endosomal protease) in a cell, or sugar linkers, for example, glucuronide-containing linkers that may be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g., linkers that are hydrolyzed when the pH is less than 5.5, such as hydrazone linkers) and linkers that are degraded under a reducing condition (e.g., disulfide-bond linkers). A non-degradable linker typically releases a drug under a condition that the antibody is hydrolyzed by a protease.

Before being linked to an antibody, a linker has a reactive group capable of reacting with certain amino acid residues, and the linkage is achieved by the reactive group. A thiol-specific reactive group is preferred, and comprises, for example, a maleimide compound, a halogenated (e.g., iodo-, bromo- or chloro-substituted) amide; a halogenated (e.g., iodo-, bromo- or chloro-substituted) ester; a halogenated (e.g., iodo-, bromo- or chloro-substituted) methyl ketone, a benzyl halide (e.g., iodide, bromide or chloride); vinyl sulfone, pyridyl disulfide; a mercury derivative such as 3,6-di-(mercurymethyl)dioxane, wherein the counter ion is acetate ion, chloride or nitrate ion; and polymethylene dimethyl sulfide thiosulfonate. The linker may include, for example, a maleimide linked to an antibody via thiosuccimide.

Drug may be any cytotoxic drug which inhibits cell growth or immunosuppression. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors. Particularly useful cytotoxic drugs include, for example, auristatins, camptothecins, duocarmycins, etoposides, maytansines and maytansinoids (e.g., DM1 and DM4), taxanes, benzodiazepines or benzodiazepine containing drugs (e.g., pyrrolo[1,4]benzodiazepines (PBDs), indolinobenzodiazepines and oxazolidinobenzodiazepines), and vinca alkaloids.

In the present invention, a drug-linker may be used to form an ADC in a simple step. In other embodiments, a bifunctional linker compound may be used to form an ADC in a two-step or multi-step process. For example, a cysteine residue is reacted with the reactive moiety of a linker in a first step, and then the functional group on the linker is reacted with a drug in the subsequent step, thereby forming an ADC.

In general, the functional group on a linker is selected so that it can specifically react with the suitable reactive group on a drug moiety. As a non-limiting example, an azide-based moiety may be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups is selected for a selective reaction between a drug moiety and a linker, each member of the complementary pair may be used for the linker, and may also be used for the drug.

The present invention also provides a method for preparing an ADC, which may further comprise: under a condition sufficient to form an antibody-drug conjugate (ADC), combining an antibody to a drug-linker compound.

In certain embodiments, the method of the present invention comprises: under a condition sufficient to form an antibody-linker conjugate, combining an antibody to a bifunctional linker compound. In these embodiments, the method of the present invention further comprises: under a condition sufficient to covalently link the drug moiety to the antibody via a linker, combining the antibody-linker conjugate to the drug moiety.

### Use

The present invention also provides uses of the antibody, antibody-drug conjugate (ADC), recombinant protein, chimeric antigen receptor (CAR) construct and/or immune cell of the present invention, for example, for preparing a diagnostic preparation or preparing a medicine.

Preferably, the medicine is used for the prevention and/or treatment of diseases related to abnormal expression or function of BCMA. The BCMA-related diseases include tumors, such as myeloma.

### Detection Use and Kit

The antibody or antibody conjugate thereof of the present invention can be used for detection, for example, for detecting a sample, thereby providing diagnostic information.

In the present invention, the sample used comprises cells, tissue samples, and biopsy specimens. The term "biopsy" as used herein shall include all kinds of biopsies known to those skilled in the art. Thus, the biopsy used in the present invention may include, for example, resected samples of tumors, tissue samples prepared by endoscopic methods, or puncture or needle biopsy of organs.

The samples used in the present invention include fixed or preserved cells or tissue samples.

The present invention also provides a kit containing the antibody (or fragment thereof) of the present invention. In a preferred embodiment of the present invention, the kit further comprises a container, instructions for use, a buffer, etc. In a preferred embodiment, the antibody of the present invention can be fixed on a detection plate.

### Pharmaceutical Composition

The present invention further provides a composition. In a preferred example, the composition is a pharmaceutical composition comprising the above single domain antibody, antibody, or fusion protein thereof, or ADC or CAR-immune cell thereof, and a pharmaceutically acceptable carrier. In general, these substances may be formulated in a non-toxic, inert and pharmaceutically acceptable aqueous carrier medium, wherein the pH is generally about 5-8, preferably, pH is about 6-8, although the pH value may be varied depending on the nature of the substances to be formulated and the condition to be treated.

The formulated pharmaceutical composition may be administered by conventional routes, including (but not limited to): intratumoral, intraperitoneal, intravenous, or topical administration. Typically, the administration route of the pharmaceutical composition of the present invention is preferably injection or oral administration. The injection administration preferably comprises intravenous injection, arterial injection, intramuscular injection, intraperitoneal injection, intradermal injection or subcutaneous injection, etc. The pharmaceutical composition of the present invention is in various conventional dosage forms, preferably in the form of solid, semi-solid or liquid, which may be an aqueous solution, a non-aqueous solution or a suspension, more preferably in the form of tablets, capsules, granules, injections or infusions.

The pharmaceutical composition according to the present invention comprises a safe and effective amount (e.g., 0.001-99 wt%, preferably 0.01-90 wt%, preferably 0.1-80 wt%) of the monoclonal antibody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 µg/kg body weight to about 5 mg/kg body weight per day. Further, the polypeptide of the present invention can also be used in combination with the other therapeutic agents.

When using the pharmaceutical composition, a safe and effective amount of the immunoconjugate is administered to a mammal, wherein the safe and effective amount is usually at least about 10 µg/kg body weight, and in most cases does not exceed about 50 mg/kg body weight, preferably the dose is about 10 µg/kg body weight to about 20 mg/kg body weight. Of course, the particular dose should also depend on various factors, such as the route of administration, patient healthy status, which are well within the skills of an experienced physician.

### The main advantages of the present invention include:

1) The single domain antibody of the present invention has specific high affinity for BCMA and can bind to human or monkey BCMA, particularly having high affinity for binding to BCMA on cell surface.
2) CAR cells comprising the single domain antibody of the present invention as an extracellular antigen-binding domain have significant tumor killing ability both *in vivo* and *in vitro*.

The present invention will be further illustrated below with reference to the specific examples. It should be understood that these examples are for illustrative purposes only and are not intended to limit the scope of the invention. The experimental methods in the following examples, in which the specific conditions are not specified, are performed under routine conditions, e.g., those described in Sambrook. et al., in Molecule Clone: A Laboratory Manual, New York: Cold Spring Harbor Laboratory Press, 1989, or as instructed by the manufacturers. Unless otherwise specified, percentages and parts are calculated by weight.

### Experimental Methods

### 1. Camel Immunization

Camel was immunized with the recombinant antigen protein following a standard 5-time immunization protocol. Human BCMA-hFC was provided by Chengdu Shengshi Junlian Biotechnology Co., Ltd.

**Table 1 Immunization Method**

| **Camel No.** | **First immunization** | **Second immunization** | **Third immunization** | **Fourth immunization** | **Fifth immunization** |
|---|---|---|---|---|---|
| **727** | Human BCMA-hFC 0.5 mg | Human BCMA-hFC 0.25 mg | Human BCMA-hFC 0.25 mg | Human BCMA-hFC 0.25 mg | Human BCMA-hFC 0.25 mg |
| **Adjuva nt** | Freund's complete adjuvant | Freund's incomplete adjuvant | Freund's incomplete adjuvant | Freund's incomplete adjuvant | Freund's incomplete adjuvant |
| **Infusio n site** | Left and right sides near cervical lymph nodes | Left and right sides near cervical lymph nodes | Left and right sides near cervical lymph nodes | Left and right sides near cervical lymph nodes | Left and right sides near cervical lymph nodes |

**Table 2 Immunization Steps**

| **Day N** | **Experiment Steps** |
|---|---|
| 0 | 5 mL of pre-immunization blood was collected, and the serum was separated for use as a negative control. |
| 0 | First immunization: the recombinant protein Human BCMA-hFC (0.5 mg) was mixed with Freund's complete adjuvant at a ratio of 1:1, emulsified, and injected on the left and right sides near the cervical lymph nodes. |
| 14 | Second immunization: the recombinant protein Human BCMA-hFC (0.25 mg) was mixed with Freund's incomplete adjuvant at a ratio of 1:1, emulsified, and injected on the left and right sides near the cervical lymph nodes. |
| 28 | Third immunization: the recombinant protein Human BCMA-hFC (0.25 mg) was mixed with Freund's incomplete adjuvant at a ratio of 1:1, emulsified, and injected on the left and right sides near the cervical lymph nodes. |
| 42 | Fourth immunization: the recombinant protein Human BCMA-hFC (0.25 mg) was mixed with Freund's incomplete adjuvant at a ratio of 1:1, emulsified, and injected on the left and right sides near the cervical lymph nodes. |
| 49 | 5 mL of peripheral blood was collected for antibody titer determination. Antibody titer determination: an ELISA plate was coated with Human BCMA-His recombinant protein, and the titers of the negative serum and the fourth immunization serum were detected. |
| 56 | Fifth immunization: the recombinant protein Human BCMA-hFC (0.25 mg) was mixed with Freund's incomplete adjuvant at a ratio of 1:1, emulsified, and injected on the left and right sides near the cervical lymph nodes. |
| 63 | 30 mL of peripheral blood was collected for antibody titer determination and nanobody library construction. Antibody titer determination: an ELISA plate was coated with Human BCMA-His recombinant protein, and the titers of the negative serum, fourth immunization serum, and fifth immunization serum were detected. |

### Camel serum titer detection:

1) Coating: Coating was performed with the antigen Human BCMA-His at a concentration of 2 µg/mL, 100 µL/well, overnight at 4°C.
2) Blocking: Blocking was performed with 1% PVA for 2 hours at room temperature (RT).
3) Sample Incubation: Camel plasma was diluted at ratios of 1:4000 to 1:128000, added at 100 µL/well, and incubated for 2 hours at RT.
4) Secondary Antibody Incubation: 100 µL/well of HRP-anti-alpaca IgG Fc was added and incubated for 1 hour at RT.
5) Color Development: 100 µL/well of TMB was added, and color was developed for 5-15 min at 37°C.
6) Termination: The reaction was terminated by adding 50 mL/well of 1 M phosphoric acid.
7) Reading: The absorbance at 450 nm was measured using a microplate reader.

### 2. Antibody Library Construction

The serum titer of the five-time immunized camel reached an ELISA titer >32000, meeting the condition for library construction. VHH3 immunization library was constructed using PBMC from the five-time immunized camel:
1) PBMCs were isolated by Ficoll density gradient centrifugation.
2) Total RNA was extracted using an RNA extraction kit (FORGENE, Cat# RE-03111).
3) cDNA was synthesized using a cDNA reverse transcription kit (TAKARA, Cat# 6210A).
4) VHH amplification: Amplification was performed using a two-step method (nested PCR).
5) Phagemid construction: The VHH fragment was inserted into the New Lib VHH XE phagemid through Gibson Assembly reaction.
6) Phage amplification: The phagemid was electroporated into supercompetent cells SR320+Helper Phage (cells prepared by Chengdu Shengshi Junlian Biotechnology Co., Ltd.).
7) Antibody library harvesting: Phage were separated and purified by PEG8000/NaCl precipitation to obtain the antibody library.

### 3. Bio panning

A Protein Panning strategy was used. First, Human BCMA-His (Chengdu Shengshi Junlian Biotechnology Co., Ltd., Cat# BCA-H522y) was used for solid-phase screening for 1-5 rounds to obtain an enriched phage library. Then, Cyno BCMA-His antigen (Chengdu Shengshi Junlian Biotechnology Co., Ltd., Cat# BCA-C52H7) was used for continued screening for 1-5 rounds to obtain a human-monkey double-positive phage enrichment library.

### 3.1 Protein Panning

1) On day 1, a Maxi-sorp plate was coated with 500 ng/well of antigen overnight at 4°C.
2) On day 2, the ELISA plate was blocked with 1% PVA for 2 hours at room temperature.
3) The antibody library was added and mixed for 2 hours at room temperature.
4) The Maxi-sorp plate was washed with PT.
5) 100 mL of 100 mM HCl was added to the Maxi-sorp plate to elute the bound phage.
6) Neutralized with 1 M Tris-HCl.
7) 100 mL of the neutralization solution was used, 1 mL of NEBalpha5F' cells (NEB, Cat# C2992I) at OD600=0.8 was infected, and shook for 1 hour at 37°C; M13K07 helper phage was added, and shook for 1 hour at 37°C; the mixture was transferred to 40 mL of 2YT/Carb/Kan medium and incubated overnight at 37°C for amplification culture.
8) Enrichment and identification: Drop method plating (performed simultaneously with step 7). On day 3, the enrichment degree was measured. PVA well was used as negative control. Enrichment was deemed successful when the number of phage clones enriched in the antigen-coated well was more than 10-fold higher than that of the phage clones enriched in the PVA well.
9) Harvesting: On day 3, the phage from the amplification culture was separated and purified using the PEG8000/NaCl precipitation method, and then the next round of panning was carried out until enrichment was successful.
10) After successful enrichment, monoclonal colonies were randomly picked for Phage ELISA.

### Phage ELISA Assay:

1) Coating: On day 1, the ELISA plate was coated with 100 ng/well of antigen recombinant protein overnight at 4°C.
2) Shaking Culture: On day 1, positive phage clones were selected and cultured in deep-well plate, and shook overnight at 37°C.
3) Blocking: On day 2, the ELISA plate was blocked with 1% PVA for 2 hours at room temperature.
4) Binding: On day 2, the deep-well plate was centrifuged, and phage was in the supernatant; 50 mL of the supernatant was moved to the ELISA plate and incubated for 2 hours at room temperature.
5) Washing: The ELISA plate was washed with PT, 100 mL of Anti-M13 HRP antibody was added, and incubated for 1 hour at room temperature.
6) Color Development: The ELISA plate was washed with PT, then washed with PBS, 100 mL of TMB was added, and color was developed for 5-10 min at 37°C.
7) Termination: 50 mL of 1 M phosphoric acid was added to terminate the reaction.
8) Measurement: The absorbance at 450 nm was measured using a microplate reader.

### 4. Antibody Expression Verification

The VHH gene fragment of the positive clone BC-HC-15 was amplified by PCR and inserted into a eukaryotic expression vector to construct a recombinant expression plasmid with a VHH-hFC structure. 293F cells were transfected for transient expression and purification to obtain the recombinant antibody protein.

### 4.1 293F Eukaryotic Transient Expression

1) Recombinant expression plasmid construction: The VHH antibody sequence was amplified by PCR and inserted into the pVHH-hIgG1Fc expression plasmid to construct a VHH-hIgG1Fc antibody recombinant expression plasmid. Correct insertion was verified by sequencing.
2) Plasmid extraction: Competent cells containing the plasmid were cultured, and the recombinant plasmid was extracted using an endotoxin-free plasmid extraction kit.
3) Transfection: The recombinant plasmid was transfected into 293F cells using a transfection reagent.
4) Expression: Expression was carried out in 50 mL of 293F cells for 5 days. The cell supernatant was collected, and the target protein was purified using protein A dextran.
5) SDS-PAGE: The purified protein was subjected to SDS-PAGE gel to identify protein purity and molecular weight.

### 4.2 ELISA Affinity Verification

The affinity of the nanobody for the antigen protein was verified by ELISA:
1) Coating: an ELISA plate was coated with 200 ng/well of the antigen protein overnight at 4°C.
2) Blocking: the ELISA plate was blocked with 1% PVA for 1.5 hours at room temperature.
3) Sample Incubation: After gradient dilution, 100 mL/well of antibodies were added to the ELISA plate and incubated for 2 hours at room temperature.
4) Secondary Antibody Incubation: The ELISA plate was washed with PT, and added with 100 mL/well of anti-human Fc HRP antibody, then incubated for 1 hour at room temperature.
5) Color Development: The ELISA plate was washed with PT, then washed with PBS, 100 mL of TMB was added, and color was developed for 5-10 min at 37°C.
6) Termination: 50 mL/well of 1 M phosphoric acid was added to terminate the reaction.
7) Measurement: The absorbance at 450 nm was measured using a microplate reader.

### 4.3 FACS Affinity Verification

The affinity of the nanobody for BCMA-positive cells was verified by FACS.
1) Cell Preparation: K562 and NCI-H929 cells were grouped at 4×10E5 cells/group, centrifuged at 300 g for 3 min, and washed 3 times with PBS.
2) Sample Incubation: 100 uL of nanobody at a final concentration of 10 µg/mL was added and incubated at 4°C for 45 min.
3) Secondary Antibody Incubation: The cells were centrifuged at 300 g for 3 min, washed 3 times with PBS, 100 µL of PE-labeled Anti-Human Fc antibody was added, and the cells were incubated at 4°C for 45 min.
4) Washing: The cells were centrifuged at 300 g for 3 min, washed 3 times with PBS, and resuspended in 300 mL of PBS per tube.
5) Flow Cytometry Detection: The fluorescence values of the cells were detected using a flow cytometer.

### Example 1 Preparation of Anti-BCMA Nanobody

### 1. Antibody Library Construction

The serum titer of the five-time immunized camel reached an ELISA titer >32000, meeting the conditions for library construction. VHH3 immune library was constructed using PBMC from the five-time immunized camel.

### 1.1. Immune Library Construction

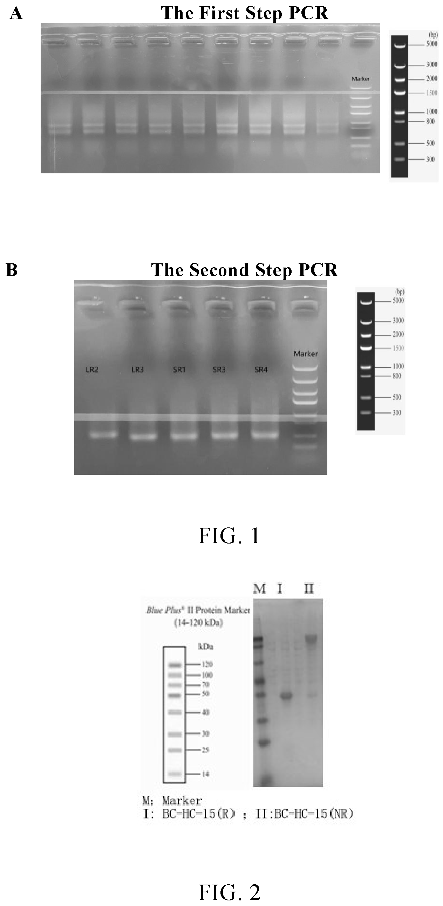

PBMCs were isolated from the peripheral blood of the five-time immunized camel. Total RNA was extracted, and then subjected to reverse transcription to obatin cDNA. Nested PCR (two-step method) was used to amplify the VHH3 gene. The first step PCR was used to amplify the gene fragment of the Leader-CH2 region. DNA electrophoresis results (Figure 1, panel A) showed: two gene fragments were obtained, of which the 700 bp fragment was the Leader-VHH-Hinge-CH2 region of the IgG2 antibody subtype; the 900 bp fragment was the Leader-VH-CH1-Hinge-CH2 region of the IgG1 antibody subtype. The 700 bp IgG2 gene fragment was recovered from the excised gel, and the VHH gene fragment was amplified by the second step PCR using specific primers. DNA electrophoresis results (Figure 1, panel B) showed: one gene fragment was obtained, approximately 400 bp in size, which was the VHH3 gene fragment of the correct length.

### 1.2. Antibody Library Quality

Using the GA reaction kit, the VHH3 gene fragment was cloned into a New Lib VHH XE vector. The GA reaction product was electroporated into supercompetent cells SR320 containing helper phage. After amplification culture, the phage was harvested, which was the immune library. Quality of the immune library: library capacity 1.40×10¹⁰, recombination efficiency (clone PCR positive clones/total clones) was 90%, meeting the requirements for the immune library. The culture supernatant was collected to obtain the antibody library (phage), cryopreserved at -80°C.

### 2. Bio panning

### 2.1. Protein Screening

The immune antibody library was screened using Human BCMA-His solid-phase screening, and obvious enrichment was obtained at round R4; then the Human BCMA-His antigen enriched library was further screened using Cyno BCMA-His antigen, and obvious enrichment was obtained at round R2, and screening was terminated (Note: if the number of clones enriched by the antigen protein (pro) / number of clones enriched by the negative control (PVA, blocking solution) >5, enrichment is considered successful).

### 2.2. Phage ELISA

A total of 96 monoclonal colonies were randomly selected from the round R2 enriched with Cyno BCMA-His antigen for phage ELISA. Positive clones were defined based on the following standard: OD value for binding to Human-P-ECD-his and Mouse-P-ECD-his recombinant protein: OD value for binding to BSA ≥2. The positive clones were subjected to gene sequencing, and the positive clone gene sequences were obtained.

**Table 3 Human-Monkey Double Positive Monoclonal Sequences from BCMA Camel Library**

| Name | Monoclonal Sequence | OD ratio (human/monkey/ PVA) |
|---|---|---|
| BC-HC-15 | EVQLVQSGGGSVQAGGSLRLSCAASGGDISS TFMGWFRQAPGKEREGVAAIYTGDGTTDYA DSVKGRFTIAQDNAKNTVYLEMSRLKPEDTA LYYCATGLDGSGSVFSPRSFRYWGQGTQVTV SS (SEQ ID NO:1) | 2.114/1.79/0.119 |

**Table 4 CDR Sequences of BC-HC-15**

| Definiti | HCDR1 | HCDR2 | HCDR3 |
|---|---|---|---|
| on Rule | (SEQ ID NO:) | (SEQ ID NO:) | (SEQ ID NO:) |
| Chothia | GGDISST (2) | YTGDGT (3) | GLDGSGSVFSPRSFRY (4) |
| Kabat | STFMG (5) | AIYTGDGTTDYADSVKG (6) | GLDGSGSVFSPRSFRY (4) |
| IMGT | GGDISSTF (7) | IYTGDGTT (8) | ATGLDGSGSVFSPRSF RY (9) |

### Example 2 Expression and Verification of Nanobody

### 1. Expression of Nanobody

The VHH gene fragment of the positive clone BC-HC-15 was amplified by PCR and inserted into a eukaryotic expression vector. 293F cells were transfected with the vector for transient expression and purification of the recombinant antibody. The SDS-PAGE electrophoresis result is shown in Figure 2. SDS-PAGE purity >90%.

### 2. ELISA Verification of Nanobody

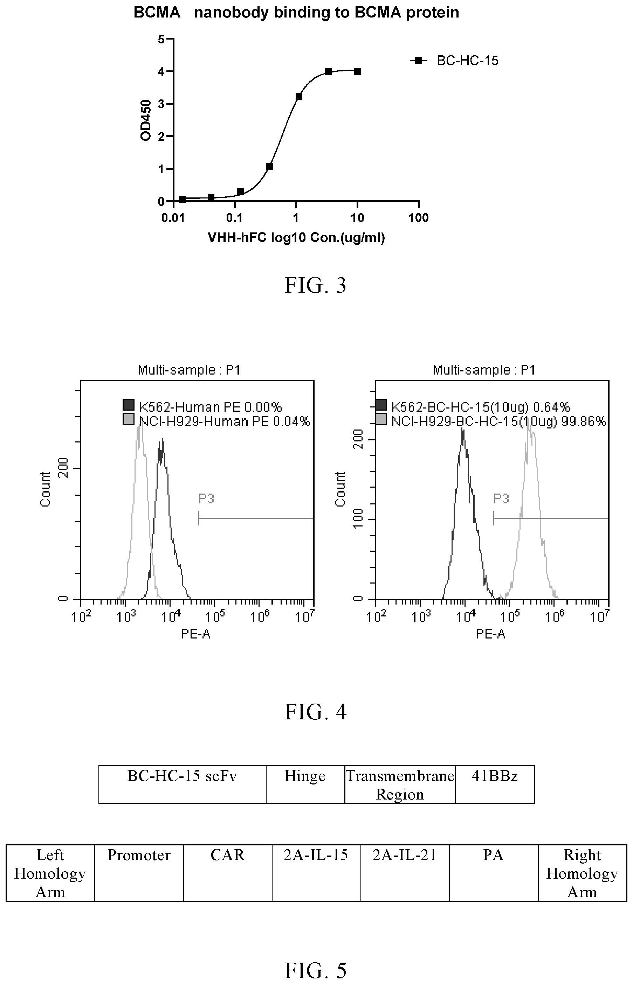

200 ng/well of Human-BCMA-His antigen protein was plated, and the affinity of the BC-HC-15 nanobody was detected by ELISA. The results showed that the affinity EC50 of the BC-HC-15 nanobody for the Human-BCMA-His antigen protein was <10 nM (Figure 3).

**Table 5. Transient Expression Data of the Preferred Antibody in Eukaryotic Cells**

| Human BCMA His (200ng/well) | |
|---|---|
| Concentration (ug/ml) | BC-HC-15 |
| 10.000 | Overflow |
| 3.333 | Overflow |
| 1.111 | 3.234 |
| 0.370 | 1.071 |
| 0.123 | 0.300 |
| 0.041 | 0.120 |
| 0.014 | 0.061 |
| 0.000 | 0.046 |
| EC50 (ug/ml) | 0.605 |
| EC50 (nM) | 7.565 |

### 3. FACS Verification of Nanobody

BCMA-negative cells K562 and BCMA-positive cells NCI-H929 were used to detect the affinity of the BC-HC-15 nanobody (10 µg/mL, 100 µL/test) by FACS. The results showed that the positive binding rate of the BC-HC-15 nanobody to NCI-H929 cells was >95% (Figure 4), indicating that the BCMA nanobody of the present invention has high affinity for binding to BCMA on cell surface.

**Table 6. Transient Expression Data of the Preferred Antibody in Eukaryotic Cells**

| Antibody ID | Antibody Structure | Concentration (µg/ml) | K562 positive Rate% | NCI-H929 Positive Rate% |
|---|---|---|---|---|
| hIgG Fc-PE | / | / | 0.00 | 0.04 |
| BC-HC-15 | VHH-hFC | 10 | 0.64 | 99.86 |

### Example 3 Construction and Expression of BC-HC-15 CAR Molecule

From N-terminus to C-terminus, the BC-HC-15 CAR molecule of this example comprises:
(1) Signal peptide (SEQ ID NO:14);
(2) BC-HC-15 nanobody;
(3) Hinge and transmembrane region (SEQ ID NO:16);
(4) Intracellular 41BB co-stimulatory signaling region and CD3ζ signal transduction sequence (SEQ ID NO:18).

To enhance the killing effect of CAR cells, the following elements are linked to the C-terminus of the BC-HC-15 CAR molecule via a T2A sequence:
(5) IL-15 (SEQ ID NO:20) and
(6) IL-21 (SEQ ID NO:22).

Sequence encoding the above molecule was constructed, with a 5' homology arm targeting the human TRAC gene (SEQ ID NO: 11) and a PGK promoter (SEQ ID NO: 23) linked to its 5' end, and a polyA sequence and a 3' homology arm (SEQ ID NO: 12) linked to its 3' end. This coding nucleic acid molecule was inserted into a minipUC-57 plasmid to obtain a plasmid encoding BCMA-CAR.

A chemically synthesized sgRNA targeting the human TRAC gene was designed (5' TCAGGGTTCTGGATATCTGT (SEQ ID NO: 27), GenScript Biotech, Nanjing). The Cas9 protein was purchased from Sino Biological (Cat# 40572-A08B).

The plasmid encoding BCMA-CAR was electroporated into human T cells activated with Dynal bead for 2-3D, knocked into the TRAC gene, and can be expressed under the control of the endogenous or exogenous promoter of the TRAC gene. Control cells were the same human T cells activated with Dynal bead for 2-3D but not electroporated.

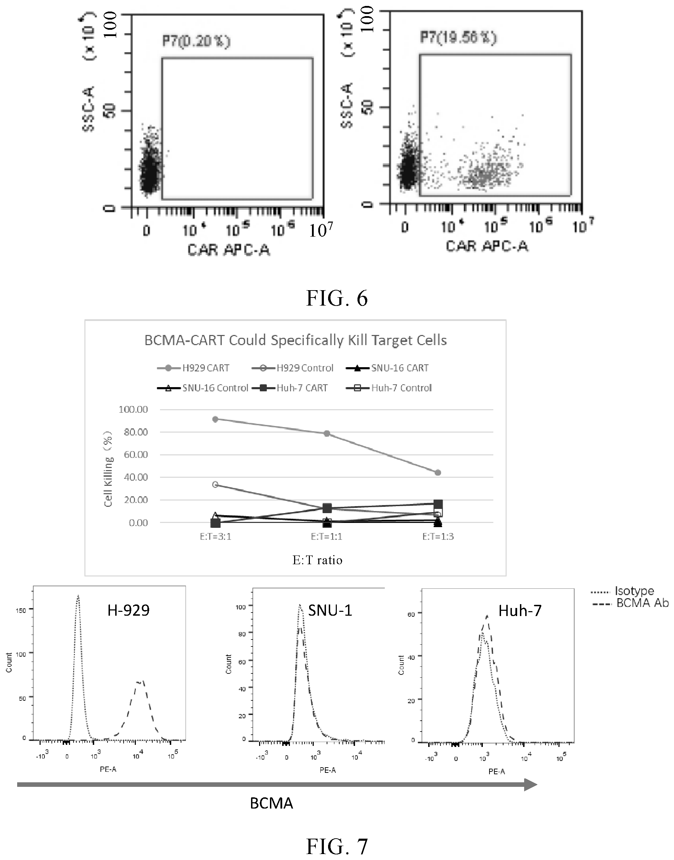

The expression level of BCMA-CAR after transfecting T cells with the BCMA-CAR plasmid was detected by FACS using fluorescently labeled BCMA protein molecules. The results are shown in Figure 6. The gating for BCMA-CAR positive cells was determined based on the positive rate of live cells in the control group being ≤1%.

### Example 4 Detection of Specific Killing Activity of BCMA-CART against Cells

To detect the specific killing ability of BCMA-CART, target cells expressing different levels of BCMA (BCMA-positive cells H929, BCMA-negative cells SNU-16, and Huh-7) stained by Calcein-AM were co-incubated overnight at effector-to-target ratios as shown in Figure 7. The number of Calcein-positive target cells was detected by flow cytometry at fixed speed and fixed time, and the target cell killing rate was calculated (Killing rate = 100% * (NC - N_trans) / NC, wherein NC = number of Calcein-positive target cells under control T cells + target cells; N_trans = number of Calcein-positive target cells under transfected BCMA-CART + target cells). The results showed that BCMA-CART could specifically kill BCMA-positive target cells.

### Example 5 Detection of BCMA-CART Expansion Stimulated by Target Cells

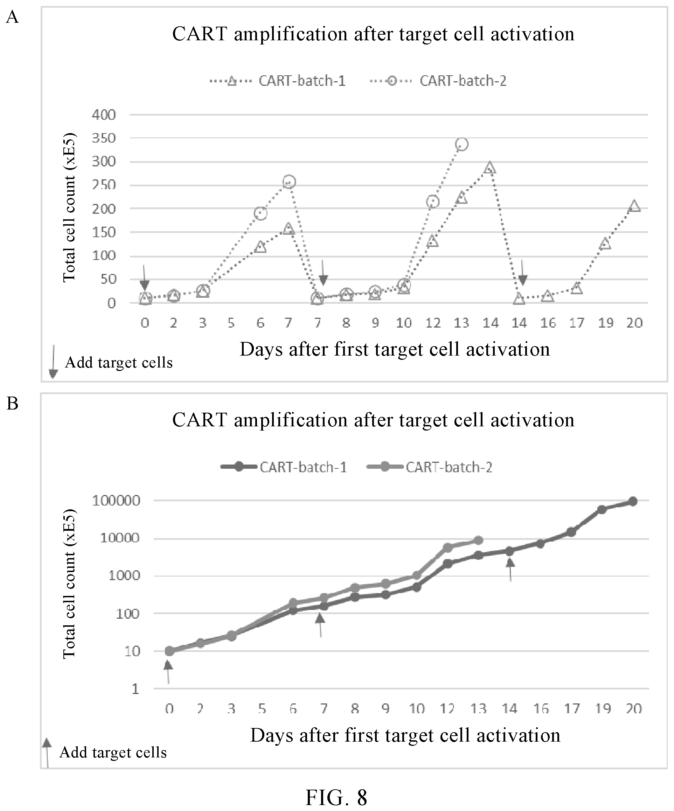

Two batches of BCMA-CART cells were prepared and cultured. As shown in Figure 8, panel A, on days 0, 7, and 14 (indicated by arrows), RPMI-8226 target cells were added at an effector-to-target ratio of 1:1 or 1:2 to activate BCMA-CART. The expansion of CART after each round of activation was counted using NC-200 at the time points indicated in Figure 8. Before each activation, CART cells were counted and adjusted to 1E6, and the number of target cells added was 1E6 or 2E6. Figure 8, panel B shows the total cell count after activation calculated from the data in Figure 8, panel A. The results show that BCMA-CART could be activated and expanded by BCMA-positive target cells.

### Example 6 In Vivo Transplantation and Antitumor Efficacy Detection of BCMA-CART Cells in Animals

NCG mice were subcutaneously implanted with RPMI-8226 multiple myeloma cells. When the tumor reached approximately 100 mm³, the mice were randomly divided into groups of 3 mice each. Approximately 2e6 revived BCMA-CART cells were injected intravenously.

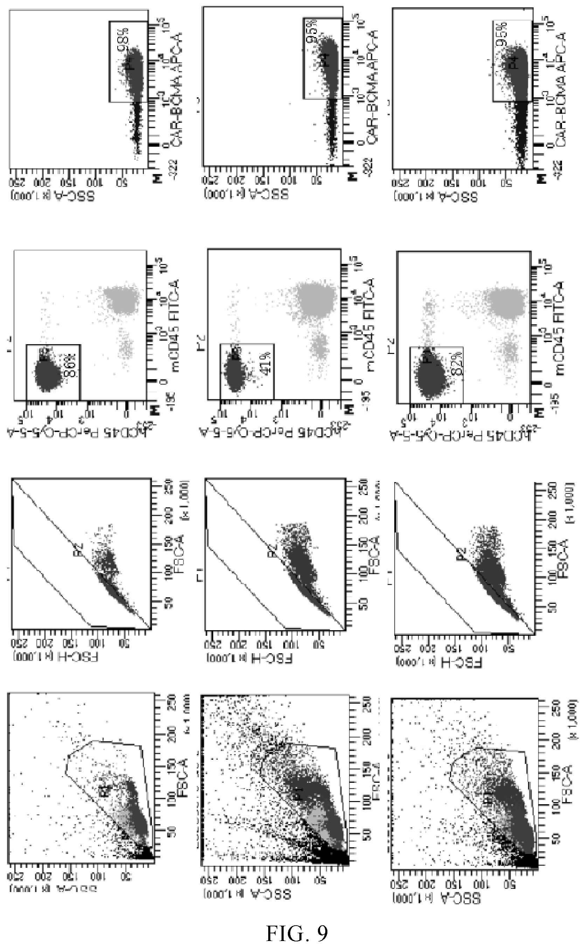

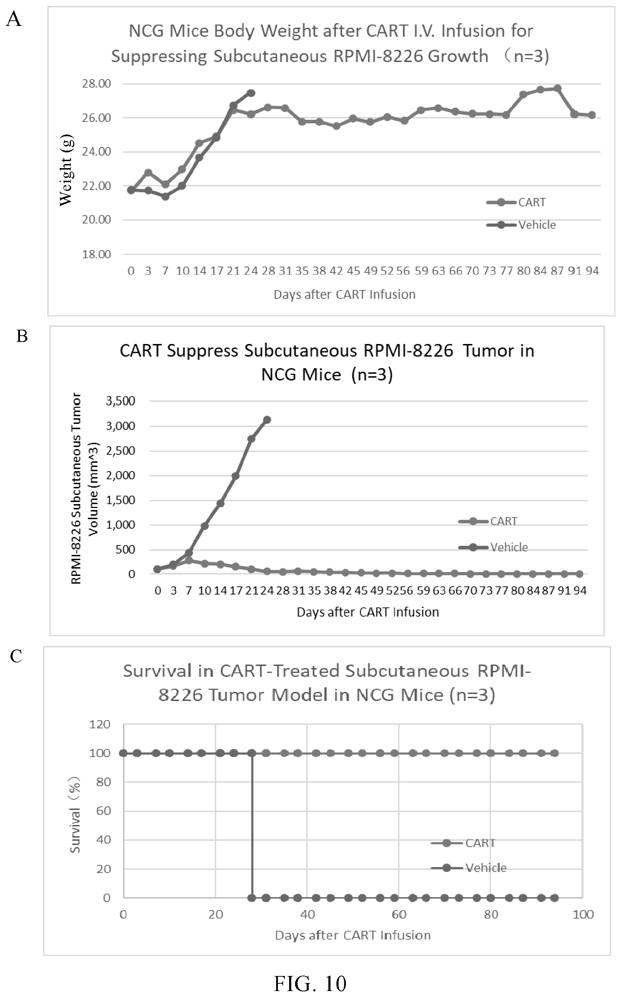

The results are shown in Figures 9 and 10. Figure 9 shows the flow cytometry analysis results of peripheral blood from three mice approximately 20 days after CART infusion, showing the proportion of human hCD45-positive human cells in the peripheral blood of the three mice, and the expression rate of BCMA-CAR in the hCD45-positive population. The results show that the CAR of the present invention has a high expression rate and is expressed in most hCD45-positive cell populations.

Figure 10, panel A shows the change of mouse body weight over time after CART infusion, indicating that the CART cells of the present invention have no significant effect on the body weight of NCG mice. Figure 10, panel B shows the killing ability of CART on tumors *in vivo*. Approximately 24 days after grouping, the tumor volume of the control group without CART infusion grew rapidly to about 3000 mm³, while the tumor volume of the CART infusion group decreased to near zero. Figure 10, panel C shows the survival rate of the control group and the CART infusion group. The CART infusion group survived until the end of the experiment on the 90th day.

The results show that the T cells of the present application can effectively kill tumors *in vivo* through intravenous infusion, demonstrating excellent anti-tumor efficacy.

### Example 7 Comparison of BC-HC VHH CAR-T with Existing BCMA-targeting CAR-T

From N-terminus to C-terminus, the G5 CAR molecule of this example comprises:
(1) Signal peptide (SEQ ID NO:14);
(2) BC-HC-15 nanobody;
(3) Hinge and transmembrane region (SEQ ID NO:16);
(4) Intracellular 41BB costimulatory signaling region and CD3ζ signal transduction sequence (SEQ ID NO:18).

The following elements are linked to the C-terminus of the BC-HC-15 CAR molecule via a 2A peptide:
(5) CD34t sequence
(6) IL-15 (SEQ ID NO:20) and
(7) IL-21 (SEQ ID NO:22).

Where CD34t (CD34 Truncated) is a truncated form of CD34, leaving only 16 amino acids in the intracellular portion, without CD34 function, but with expression of the extracellular region of CD34 on the membrane surface for enrichment and detection. The positive control G2 CAR has the same structure as G5 CAR, except that the BC-HC-15 nanobody was replaced by the scFv of the existing antibody BCMA-50, whose heavy chain variable region and light chain variable region sequences are shown in SEQ ID NO: 11 and 12 of US2017/0183418A1. The negative control G7 is an empty vector.

An exogenous EF1a promoter (SEQ ID NO: 26) was linked to the 5' end of the CAR molecule coding sequence. Plasmids were constructed and introduced into T cells using the method described in the above examples to obtain CAR-T cells expressing G5 CAR. According to the method described in Example 6, *in vivo* anti-tumor efficacy was tested on the RPMI-1640 subcutaneous tumor-bearing animal model. Each animal received a single intravenous infusion of 2.5E6 CART.

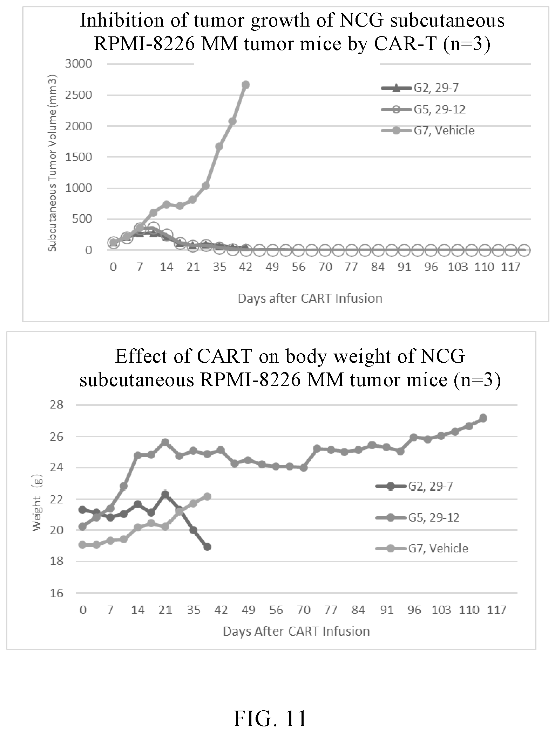

The results are shown in Figure 11. Both the positive control G2 CAR and the G5 CAR of the present invention showed significant tumor inhibition efficacy, almost completely suppressing subcutaneous tumors. Among them, G5 CAR achieved long-term tumor suppression reaching the 120D endpoint of the experiment, while in the positive control G2 CAR group, all animals died before the planned endpoint of the experiment around 42D. The animal body weight also reflected that the body weight of the G2 CAR group began to drop sharply from about 20D, consistent with animal death around 42D, while the body weight of the G5 CAR group was steady and increased during the 120D planned experiment period. Flow cytometry analysis of peripheral blood on day 21 after CART infusion showed that the proportion of CART in the humanized hCD45 population was between 20-40% for both G5 and G2, but G5 had better cell engraftment than G2. This result suggests that the CAR-T cell of the present invention has both efficient tumor inhibition efficacy and good safety.

The nucleotide sequence encoding the BC-HC-15 CAR-IL15-IL21 molecule is shown below (including homology arms): 
CAGTTTGCTTTGCTGGGCCTTTTTCCCATGCCTGCCTTTACTCTGCCA
GAGTTATATTGCTGGGGTTTTGAAGAAGATCCTATTAAATAAAAGAATAA
GCAGTATTATTAAGTAGCCCTGCATTTCAGGTTTCCTTGAGTGGCAGGCC
AGGCCTGGCCGTGAACGTTCACTGAAATCATGGCCTCTTGGCCAAGATTG
ATAGCTTGTGCCTGTCCCTGAGTCCCAGTCCATCACGAGCAGCTGGTTTCT
AAGATGCTATTTCCCGTATAAAGCATGAGACCGTGACTTGCCAGCCCCAC
AGAGCCCCGCCCTTGTCCATCACTGGCATCTGGACTCCAGCCTGGGTTGG
GGCAAAGAGGGAAATGAGATCATGTCCTAACCCTGATCCTCTTGTCCCAC
ATTGGGGTTGCGCCTTTTCCAAGGCAGCCCTGGGTTTGCGCAGGGACGCG
GCTGCTCTGGGCGTGGTTCCGGGAAACGCAGCGGCGCCGACCCTGGGTCT
CGCACATTCTTCACGTCCGTTCGCAGCGTCACCCGGATCTTCGCCGCTACC
CTTGTGGGCCCCCCGGCGACGCTTCCTGCTCCGCCCCTAAGTCGGGAAGG
TTCCTTGCGGTTCGCGGCGTGCCGGACGTGACAAACGGAAGCCGCACGTC
TCACTAGTACCCTCGCAGACGGACAGCGCCAGGGAGCAATGGCAGCGCG
CCGACCGCGATGGGCTGTGGCCAATAGCGGCTGCTCAGCAGGGCGCGCC
GAGAGCAGCGGCCGGGAAGGGGCGGTGCGGGAGGCGGGGTGTGGGGCG
GTAGTGTGGGCCCTGTTCCTGCCCGCGCGGTGTTCCGCATTCTGCAAGCCT
CCGGAGCGCACGTCGGCAGTCGGCTCCCTCGTTGACCGAATCACCGACCT
CTCTCCCCAGGGGGATCATGGCCTTACCAGTGACCGCCTTGCTCCTGCCG
CTGGCCTTGCTGCTCCACGCCGCCAGGCCGGAAGTACAGCTCGTGCAGTC
TGGGGGAGGCTCGGTGCAGGCTGGAGGGTCTCTGAGACTCTCCTGCGCTG
 
CCTCTGGAGGTGATATCAGTAGCACCTTCATGGGCTGGTTCCGCCAGGCT
CCAGGTAAGGAGCGCGAGGGGGTCGCAGCAATTTATACTGGTGACGGCA
CCACCGACTATGCCGACTCCGTGAAGGGCCGATTCACCATCGCCCAAGAC
AACGCCAAGAACACGGTGTATCTCGAAATGAGTCGTCTGAAACCTGAGG
ACACTGCCTTGTACTACTGTGCGACAGGCCTGGACGGCAGCGGTAGCGTC
TTTTCACCCCGGAGCTTTCGTTACTGGGGCCAAGGGACCCAGGTCACCGT
CTCCTCAACCACGACGCCAGCGCCGCGACCACCAACACCGGCGCCCACC
ATCGCGTCGCAGCCCCTGTCCCTGCGCCCAGAGGCGTGCCGGCCAGCGGC
GGGGGGCGCAGTGCACACGAGGGGGCTGGACTTCGCCTGTGATATCTAC
ATCTGGGCGCCCTTGGCCGGGACTTGTGGGGTCCTTCTCCTGTCACTGGTT
ATCACCCTTTACTGCAAACGGGGCAGAAAGAAACTCCTGTATATATTCAA
ACAACCATTTATGAGACCAGTACAAACTACTCAAGAGGAAGATGGCTGT
AGCTGCCGATTTCCAGAAGAAGAAGAAGGAGGATGTGAACTGAGAGTGA
AGTTCAGCAGGAGCGCAGACGCCCCCGCGTACCAGCAGGGCCAGAACCA
GCTCTATAACGAGCTCAATCTAGGACGAAGAGAGGAGTACGATGTTTTGG
ACAAGAGACGTGGCCGGGACCCTGAGATGGGGGGAAAGCCGAGAAGGA
AGAACCCTCAGGAAGGCCTGTACAATGAACTGCAGAAAGATAAGATGGC
GGAGGCCTACAGTGAGATTGGGATGAAAGGCGAGCGCCGGAGGGGCAAG
GGGCACGATGGCCTTTACCAGGGTCTCAGTACAGCCACCAAGGACACCTA
CGACGCCCTTCACATGCAGGCCCTGCCCCCTCGCGGTTCTGGCGTGAAAC
AGACTTTGAATTTTGACCTTCTCAAGTTGGCGGGAGACGTGGAGTCCAAC
CCAGGGCCCATGAGAATTTCGAAACCACATTTGAGAAGTATTTCCATCCA
GTGCTACTTGTGTTTACTTCTAAACAGTCATTTTCTAACTGAAGCTGGCAT
TCATGTCTTCATTTTGGGCTGTTTCAGTGCAGGGCTTCCTAAAACAGAAGC
CAACTGGGTGAATGTAATAAGTGATTTGAAAAAAATTGAAGATCTTATTC
AATCTATGCATATTGATGCTACTTTATATACGGAAAGTGATGTTCACCCC
AGTTGCAAAGTAACAGCAATGAAGTGCTTTCTCTTGGAGTTACAAGTTAT
TTCACTTGAGTCCGGAGATGCAAGTATTCATGATACAGTAGAAAATCTGA
TCATCCTAGCAAACAACAGTTTGTCTTCTAATGGGAATGTAACAGAATCT
GGATGCAAAGAATGTGAGGAACTGGAGGAAAAAAATATTAAAGAATTTT
TGCAGAGTTTTGTACATATTGTCCAAATGTTCATCAACACTTCTGGAAGC
GGACAGTGTACTAATTATGCTCTCTTGAAATTGGCTGGAGATGTTGAGAG
CAACCCTGGACCTATGAGATCCAGTCCTGGCAACATGGAGAGGATTGTCA
TCTGTCTGATGGTCATCTTCTTGGGGACACTGGTCCACAAATCAAGCTCCC
AAGGTCAAGATCGCCACATGATTAGAATGCGTCAACTTATAGATATTGTT
 
GATCAGCTGAAAAATTATGTGAATGACTTGGTCCCTGAATTTCTGCCAGC
TCCAGAAGATGTAGAGACAAACTGTGAGTGGTCAGCTTTTTCCTGCTTTC
AGAAGGCCCAACTAAAGTCAGCAAATACAGGAAACAATGAAAGGATAAT
CAATGTATCAATTAAAAAGCTGAAGAGGAAACCACCTTCCACAAATGCA
GGGAGAAGACAGAAACACAGACTAACATGCCCTTCATGTGATTCTTATGA
GAAAAAACCACCCAAAGAATTCCTAGAAAGATTCAAATCACTTCTCCAAA
AGATGATTCATCAGCATCTGTCCTCTAGAACACACGGAAGTGAAGATTCC
TGACTGTGCCTTCTAGTTGCCAGCCATCTGTTGTTTGCCCCTCCCCCGTGC
CTTCCTTGACCCTGGAAGGTGCCACTCCCACTGTCCTTTCCTAATAAAATG
AGGAAATTGCATCGCATTGTCTGAGTAGGTGTCATTCTATTCTGGGGGGT
GGGGTGGGGCAGGACAGCAAGGGGGAGGATTGGGAAGACAATAGCAGG
CATGCTGGGGATGCGGTGGGCTCTATGGGAATTCTCTAGAAATATTCTCG
AGGTTTAAACGTCGACGCGGCCGCGATATCCAGAACCCTGACCCTGCCGT
GTACCAGCTGAGAGACTCTAAATCCAGTGACAAGTCTGTCTGCCTATTCA
CCGATTTTGATTCTCAAACAAATGTGTCACAAAGTAAGGATTCTGATGTG
TATATCACAGACAAAACTGTGCTAGACATGAGGTCTATGGACTTCAAGAG
CAACAGTGCTGTGGCCTGGAGCAACAAATCTGACTTTGCATGTGCAAACG
CCTTCAACAACAGCATTATTCCAGAAGACACCTTCTTCCCCAGCCCAGGT
AAGGGCAGCTTTGGTGCCTTCGCAGGCTGTTTCCTTGCTTCAGGAATGGC
CAGGTTCTGCCCAGAGCTCTGGTCAATGATGTCTAAAACTCCTCTGATTG
GTGGTCTCGGCCTTATCCATTGCC (SEQ ID NO:10)
Left homology arm sequence (SEQ ID NO:11): 
CAGTTTGCTTTGCTGGGCCTTTTTCCCATGCCTGCCTTTACTCTGCCA
GAGTTATATTGCTGGGGTTTTGAAGAAGATCCTATTAAATAAAAGAATAA
GCAGTATTATTAAGTAGCCCTGCATTTCAGGTTTCCTTGAGTGGCAGGCC
AGGCCTGGCCGTGAACGTTCACTGAAATCATGGCCTCTTGGCCAAGATTG
ATAGCTTGTGCCTGTCCCTGAGTCCCAGTCCATCACGAGCAGCTGGTTTCT
AAGATGCTATTTCCCGTATAAAGCATGAGACCGTGACTTGCCAGCCCCAC
AGAGCCCCGCCCTTGTCCATCACTGGCATCTGGACTCCAGCCTGGGTTGG
GGCAAAGAGGGAAATGAGATCATGTCCTAACCCTGATCCTCTTGTCCCAC
A
Right homology arm sequence (SEQ ID NO:12): 
GATATCCAGAACCCTGACCCTGCCGTGTACCAGCTGAGAGACTCTAA
ATCCAGTGACAAGTCTGTCTGCCTATTCACCGATTTTGATTCTCAAACAAA
 
TGTGTCACAAAGTAAGGATTCTGATGTGTATATCACAGACAAAACTGTGC
TAGACATGAGGTCTATGGACTTCAAGAGCAACAGTGCTGTGGCCTGGAGC
AACAAATCTGACTTTGCATGTGCAAACGCCTTCAACAACAGCATTATTCC
AGAAGACACCTTCTTCCCCAGCCCAGGTAAGGGCAGCTTTGGTGCCTTCG
CAGGCTGTTTCCTTGCTTCAGGAATGGCCAGGTTCTGCCCAGAGCTCTGG
TCAATGATGTCTAAAACTCCTCTGATTGGTGGTCTCGGCCTTATCCATTGC
C
Signal peptide nucleic acid sequence (SEQ ID NO:13): Atggccttaccagtgaccgccttgctcctgccgctggccttgctgctccacgccgccaggccg
Signal peptide amino acid sequence (SEQ ID NO:14):
   MALPVTALLLPLALLLHAARP
Hinge-transmembrane nucleic acid sequence (SEQ ID NO:15): 
ACCACGACGCCAGCGCCGCGACCACCAACACCGGCGCCCACCATCG
CGTCGCAGCCCCTGTCCCTGCGCCCAGAGGCGTGCCGGCCAGCGGCGGGG
GGCGCAGTGCACACGAGGGGGCTGGACTTCGCCTGTGATATCTACATCTG
GGCGCCCTTGGCCGGGACTTGTGGGGTCCTTCTCCTGTCACTGGTTATCAC
CCTTTACTGC
Hinge-transmembrane amino acid sequence (SEQ ID NO:16): 
TTTPAPRPPTPAPTIASQPLSLRPEACRPAAGGAVHTRGLDFACDIYIWA
PLAGTCGVLLLSLVITLYC
41BBz nucleic acid sequence (SEQ ID NO:17): 
AAACGGGGCAGAAAGAAACTCCTGTATATATTCAAACAACCATTTAT
GAGACCAGTACAAACTACTCAAGAGGAAGATGGCTGTAGCTGCCGATTTC
CAGAAGAAGAAGAAGGAGGATGTGAACTGAGAGTGAAGTTCAGCAGGA
GCGCAGACGCCCCCGCGTACCAGCAGGGCCAGAACCAGCTCTATAACGA
GCTCAATCTAGGACGAAGAGAGGAGTACGATGTTTTGGACAAGAGACGT
GGCCGGGACCCTGAGATGGGGGGAAAGCCGAGAAGGAAGAACCCTCAG
GAAGGCCTGTACAATGAACTGCAGAAAGATAAGATGGCGGAGGCCTACA
GTGAGATTGGGATGAAAGGCGAGCGCCGGAGGGGCAAGGGGCACGATG
GCCTTTACCAGGGTCTCAGTACAGCCACCAAGGACACCTACGACGCCCTT
CACATGCAGGCCCTGCCCCCTCGC
41BBz amino acid sequence (SEQ ID NO:18): 
KRGRKKLLYIFKQPFMRPVQTTQEEDGCSCRFPEEEEGGCELRVKFSRS
ADAPAYQQGQNQLYNELNLGRREEYDVLDKRRGRDPEMGGKPRRKNPQEG
LYNELQKDKMAEAYSEIGMKGERRRGKGHDGLYQGLSTATKDTYDALHMQ
 ALPPR
IL-15 nucleic acid sequence (SEQ ID NO:19): 
ATGAGAATTTCGAAACCACATTTGAGAAGTATTTCCATCCAGTGCTA
CTTGTGTTTACTTCTAAACAGTCATTTTCTAACTGAAGCTGGCATTCATGT
CTTCATTTTGGGCTGTTTCAGTGCAGGGCTTCCTAAAACAGAAGCCAACT
GGGTGAATGTAATAAGTGATTTGAAAAAAATTGAAGATCTTATTCAATCT
ATGCATATTGATGCTACTTTATATACGGAAAGTGATGTTCACCCCAGTTG
CAAAGTAACAGCAATGAAGTGCTTTCTCTTGGAGTTACAAGTTATTTCAC
TTGAGTCCGGAGATGCAAGTATTCATGATACAGTAGAAAATCTGATCATC
CTAGCAAACAACAGTTTGTCTTCTAATGGGAATGTAACAGAATCTGGATG
CAAAGAATGTGAGGAACTGGAGGAAAAAAATATTAAAGAATTTTTGCAG
AGTTTTGTACATATTGTCCAAATGTTCATCAACACTTCTTGA
IL-15 amino acid sequence (SEQ ID NO:20): 
MRISKPHLRSISIQCYLCLLLNSHFLTEAGIHVFILGCFSAGLPKTEANWV
NVISDLKKIEDLIQSMHIDATLYTESDVHPSCKVTAMKCFLLELQVISLESGD
ASIHDTVENLIILANNSLSSNGNVTESGCKECEELEEKNIKEFLQSFVHIVQMFI
NTS-
IL-21 nucleic acid sequence (SEQ ID NO:21): 
ATGAGATCCAGTCCTGGCAACATGGAGAGGATTGTCATCTGTCTGAT
GGTCATCTTCTTGGGGACACTGGTCCACAAATCAAGCTCCCAAGGTCAAG
ATCGCCACATGATTAGAATGCGTCAACTTATAGATATTGTTGATCAGCTG
AAAAATTATGTGAATGACTTGGTCCCTGAATTTCTGCCAGCTCCAGAAGA
TGTAGAGACAAACTGTGAGTGGTCAGCTTTTTCCTGCTTTCAGAAGGCCC
AACTAAAGTCAGCAAATACAGGAAACAATGAAAGGATAATCAATGTATC
AATTAAAAAGCTGAAGAGGAAACCACCTTCCACAAATGCAGGGAGAAGA
CAGAAACACAGACTAACATGCCCTTCATGTGATTCTTATGAGAAAAAACC
ACCCAAAGAATTCCTAGAAAGATTCAAATCACTTCTCCAAAAGATGATTC
ATCAGCATCTGTCCTCTAGAACACACGGAAGTGAAGATTCCTGA
IL-21 amino acid sequence (SEQ ID NO:22): 
MRSSPGNMERIVICLMVIFLGTLVHKSSSQGQDRHMIRMRQLIDIVDQL
KNYVNDLVPEFLPAPEDVETNCEWSAFSCFQKAQLKSANTGNNERIINVSIK
KLKRKPPSTNAGRRQKHRLTCPSCDSYEKKPPKEFLERFKSLLQKMIHQHLSS
RTHGSEDS-
PGK sequence (SEQ ID NO:23): Ttggggttgcgccttttccaaggcagccctgggtttgcgcagggacgcggctgctctgggcgtggttccggga 
aacgcagcggcgccgaccctgggtctcgcacattcttcacgtccgttcgcagcgtcacccggatcttcgccgctaccct
tgtgggccccccggcgacgcttcctgctccgcccctaagtcgggaaggttccttgcggttcgcggcgtgccggacgtg
acaaacggaagccgcacgtctcactagtaccctcgcagacggacagcgccagggagcaatggcagcgcgccgacc
gcgatgggctgtggccaatagcggctgctcagcagggcgcgccgagagcagcggccgggaaggggcggtgcggg
aggcggggtgtggggcggtagtgtgggccctgttcctgcccgcgcggtgttccgcattctgcaagcctccggagcgc
acgtcggcagtcggctccctcgttgaccgaatcaccgacctctctccccagggggatc
BC-HC-15 CAR (BC-HC-15 VHH-Hinge-TM-41BBz) nucleic acid sequence (SEQ ID NO:24): 
GAAGTACAGCTCGTGCAGTCTGGGGGAGGCTCGGTGCAGGCTGGAG
GGTCTCTGAGACTCTCCTGCGCTGCCTCTGGAGGTGATATCAGTAGCACC
TTCATGGGCTGGTTCCGCCAGGCTCCAGGTAAGGAGCGCGAGGGGGTCGC
AGCAATTTATACTGGTGACGGCACCACCGACTATGCCGACTCCGTGAAGG
GCCGATTCACCATCGCCCAAGACAACGCCAAGAACACGGTGTATCTCGAA
ATGAGTCGTCTGAAACCTGAGGACACTGCCTTGTACTACTGTGCGACAGG
CCTGGACGGCAGCGGTAGCGTCTTTTCACCCCGGAGCTTTCGTTACTGGG
GCCAAGGGACCCAGGTCACCGTCTCCTCAACCACGACGCCAGCGCCGCG
ACCACCAACACCGGCGCCCACCATCGCGTCGCAGCCCCTGTCCCTGCGCC
CAGAGGCGTGCCGGCCAGCGGCGGGGGGCGCAGTGCACACGAGGGGGCT
GGACTTCGCCTGTGATATCTACATCTGGGCGCCCTTGGCCGGGACTTGTG
GGGTCCTTCTCCTGTCACTGGTTATCACCCTTTACTGCAAACGGGGCAGA
AAGAAACTCCTGTATATATTCAAACAACCATTTATGAGACCAGTACAAAC
TACTCAAGAGGAAGATGGCTGTAGCTGCCGATTTCCAGAAGAAGAAGAA
GGAGGATGTGAACTGAGAGTGAAGTTCAGCAGGAGCGCAGACGCCCCCG
CGTACCAGCAGGGCCAGAACCAGCTCTATAACGAGCTCAATCTAGGACG
AAGAGAGGAGTACGATGTTTTGGACAAGAGACGTGGCCGGGACCCTGAG
ATGGGGGGAAAGCCGAGAAGGAAGAACCCTCAGGAAGGCCTGTACAATG
AACTGCAGAAAGATAAGATGGCGGAGGCCTACAGTGAGATTGGGATGAA
AGGCGAGCGCCGGAGGGGCAAGGGGCACGATGGCCTTTACCAGGGTCTC
AGTACAGCCACCAAGGACACCTACGACGCCCTTCACATGCAGGCCCTGCC
CCCTCGC
BC-HC-15 CAR (BC-HC-15 VHH-Hinge-TM-41BBz) amino acid sequence (SEQ ID NO:25): EVQLVQSGGGSVQAGGSLRLSCAASGGDISSTFMGWFRQAPGKEREGV 
AAIYTGDGTTDYADSVKGRFTIAQDNAKNTVYLEMSRLKPEDTALYYCATG
LDGSGSVFSPRSFRYWGQGTQVTVSSTTTPAPRPPTPAPTIASQPLSLRPEACR
PAAGGAVHTRGLDFACDIYIWAPLAGTCGVLLLSLVITLYCKRGRKKLLYIF
KQPFMRPVQTTQEEDGCSCRFPEEEEGGCELRVKFSRSADAPAYQQGQNQL
YNELNLGRREEYDVLDKRRGRDPEMGGKPRRKNPQEGLYNELQKDKMAEA
YSEIGMKGERRRGKGHDGLYQGLSTATKDTYDALHMQALPPR
EF1a promoter sequence (SEQ ID NO:26):

Ggctccggtgcccgtcagtgggcagagcgcacatcgcccacagtccccgagaagttggggggaggggtcg
gcaattgaaccggtgcctagagaaggtggcgcggggtaaactgggaaagtgatgtcgtgtactggctccgcctttttcc
cgagggtgggggagaaccgtatataagtgcagtagtcgccgtgaacgttctttttcgcaacgggtttgccgccagaaca
caggtaagtgccgtgtgtggttcccgcgggcctggcctctttacgggttatggcccttgcgtgccttgaattacttccacct
ggctgcagtacgtgattcttgatcccgagcttcgggttggaagtgggtgggagagttcgaggccttgcgcttaaggagc
cccttcgcctcgtgcttgagttgaggcctggcctgggcgctggggccgccgcgtgcgaatctggtggcaccttcgcgc
ctgtctcgctgctttcgataagtctctagccatttaaaatttttgatgacctgctgcgacgctttttttctggcaagatagtcttg
taaatgcgggccaagatctgcacactggtatttcggtttttggggccgcgggcggcgacggggcccgtgcgtcccagc
gcacatgttcggcgaggcggggcctgcgagcgcggccaccgagaatcggacgggggtagtctcaagctggccggc
ctgctctggtgcctggcctcgcgccgccgtgtatcgccccgccctgggcggcaaggctggcccggtcggcaccagtt
gcgtgagcggaaagatggccgcttcccggccctgctgcagggagctcaaaatggaggacgcggcgctcgggagag
cgggcgggtgagtcacccacacaaaggaaaagggcctttccgtcctcagccgtcgcttcatgtgactccacggagtac
cgggcgccgtccaggcacctcgattagttctcgagcttttggagtacgtcgtctttaggttggggggaggggttttatgcg
atggagtttccccacactgagtgggtggagactgaagttaggccagcttggcacttgatgtaattctccttggaatttgcc
ctttttgagtttggatcttggttcattctcaagcctcagacagtggttcaaagtttttttcttccatttcaggtgtcgtga

All documents mentioned in the present application are incorporated by reference in this application, as if each document is individually incorporated by reference. In addition, it should be understood that, after reading the above teachings of the present invention, those skilled in the art can make various changes or modifications, and these equivalents also fall within the scope of the claims as defined in the appended claims.

## Claims

1. An anti-BCMA single domain antibody, wherein the VHH chain of the single domain antibody has complementarity determining regions CDR1, CDR2, and CDR3 of the heavy chain variable region as set forth in SEQ ID NO: 1, and the complementarity determining regions CDR1, CDR2, and CDR3 are defined by Chothia, Abm, Kabat, or IMGT rule.

2. The single domain antibody of claim 1, wherein the CDR1, CDR2, and CDR3 are selected from the group consisting of:
(1) on the basis of Chothia rule:
CDR1 as set forth in SEQ ID NO: 2,
CDR2 as set forth in SEQ ID NO: 3, and
CDR3 as set forth in SEQ ID NO: 4; or,
(2) on the basis of Kabat rule:
CDR1 as set forth in SEQ ID NO: 5,
CDR2 as set forth in SEQ ID NO: 6, and
CDR3 as set forth in SEQ ID NO: 4; or,
(3) on the basis of IMGT rule:
CDR1 as set forth in SEQ ID NO: 7,
CDR2 as set forth in SEQ ID NO: 8, and
CDR3 as set forth in SEQ ID NO: 9.

3. The single domain antibody of claim 1, wherein the VHH chain of the single domain antibody has an amino acid sequence having ≥65%, ≥75%, ≥80%, ≥85%, ≥90%, ≥95%, ≥96%, ≥97%, ≥98%, or ≥99% sequence identity to the amino acid sequence as set forth in SEQ ID NO: 1, or has an amino acid sequence as set forth in SEQ ID NO: 1.

4. An anti-BCMA antibody, wherein the antibody comprises one or more VHH chains of the single domain antibody of claim 1.

5. A multispecific antibody, wherein the multispecific antibody comprises: the single domain antibody of claim 1 or the anti-BCMA antibody of claim 4.

6. A recombinant protein, wherein the recombinant protein comprises:
(i) the single domain antibody of claim 1, or the anti-BCMA antibody of claim 4; and
(ii) an optional polypeptide molecule or fragment with therapeutic function; and/or
(iii) an optional functional domain for improving the physicochemical property or druggability of the protein.

7. A chimeric antigen receptor (CAR), wherein the nucleotide sequence of the extracellular antigen-binding domain of the CAR encoding the VHH chain of the single domain antibody of claim 1.

8. The CAR of claim 7, wherein the VHH chain of the single domain antibody has an amino acid sequence having ≥65%, ≥75%, ≥80%, ≥85%, ≥90%, ≥95%, ≥96%, ≥97%, ≥98%, or ≥99% sequence identity to the amino acid sequence as set forth in SEQ ID NO: 1, or has an amino acid sequence as set forth in SEQ ID NO: 1.

9. The CAR of claim 7, wherein the protein sequence encoded by the CAR nucleotide sequence has a structure as shown in Formula I below:
L-EB-H-TM-Cₙ-IS (I)
wherein,
each "-" independently is a linker peptide or a peptide bond;
L is absent or a signal peptide sequence;
EB is the extracellular antigen-binding domain;
H is absent or a hinge region;
TM is a transmembrane domain;
Cₙ is n tandem co-stimulatory signaling molecules, wherein C represents a co-stimulatory signaling molecule, and n is an integer selected from 1, 2, 3, or 4;
IS is a cytoplasmic signal transduction sequence.

10. A polynucleotide, wherein the polynucleotide encodes a polypeptide selected from the group consisting of:
(1) the single domain antibody of claim 1, the anti-BCMA antibody of claim 4, or the multispecific antibody of claim 5;
(2) the recombinant protein of claim 6; or
(3) the CAR of claim 7.

11. The polynucleotide of claim 10, wherein the nucleic acid molecule encoding the CAR has a structure of Formula Ia:
ARM5-P1-CAR1-P2/L1-Z1-P3/L2-Z2-(P4/L3-Z3)ₘ-ARM3 (Ia)
wherein,
ARM5 is absent or a 5' homology arm;
ARM3 is absent or a 3' homology arm;
P1 is absent of promoter, a splicing acceptor, or a first exogenous promoter;
CAR1 is a nucleic acid sequence encoding the chimeric antigen receptor (CAR) of claim 7;
P2/L1 is a second exogenous promoter P2 or a nucleic acid sequence L1 encoding a cleavable moiety;
Z1 is absent, or a nucleotide sequence encoding a second CAR, or IL-15 or a functional protein fragment thereof;
P3/L2 is absent or a third exogenous promoter P3 or a nucleic acid sequence L2 encoding a cleavable moiety;
Z2 is absent, or a nucleotide sequence encoding IL-21 or a functional protein fragment thereof;
P4/L3 is each independently a fourth exogenous promoter P4 or a nucleic acid sequence L3 encoding a cleavable moiety;
Z3 is a nucleic acid sequence encoding an additional functional protein;
m is 0, 1, 2, 3, 4 or 5.

12. The polynucleotide of claim 11, wherein the promoters P1, P2, P3, and each P4 are the same or different exogenous promoters, and the cleavable moieties L1, L2, and L3 are nucleic acid sequences encoding the same or different cleavable moieties.

13. The polynucleotide of claim 11, wherein the polynucleotide has a nucleotide sequence as set forth in SEQ ID NO: 10.

14. The polynucleotide of claim 11, wherein the functional protein is selected from the group consisting of: IL-21 or a functionally active fragment thereof, IL-15 or a functionally active fragment thereof, IL-2 or a functionally active fragment thereof, IL-7 or a functionally active fragment thereof, IL-10 or a functional fragment thereof, a cytokine, an immune blocker targeting PD1, TIGIT, VGEF, CTLA-4, CD73, an antibody, a BiTE, and a combination thereof.

15. A vector, wherein the vector comprises the polynucleotide of claim 10.

16. The vector of claim 15, wherein the vector is a plasmid.

17. A recombinant immune cell, wherein the immune cell exogenously expresses the CAR of claim 7, comprises the vector of claim 15, or has the polynucleotide of claim 10 integrated into its genome.

18. The recombinant immune cell of claim 17, wherein the polynucleotide is introduced into the immune cell by plasmid electroporation method.

19. The recombinant immune cell of claim 18, wherein the polynucleotide is site-specifically integrated into the DNA of the immune cell by plasmid electroporation and a site-directed nuclease method.

20. The recombinant immune cell of claim 19, wherein the site-directed nuclease comprises a CRISPR-associated protein and an sgRNA.

21. The recombinant immune cell of claim 20, wherein the site-directed nuclease comprises an sgRNA targeting TRAC gene.
